(19) Europäisches Patentamt European Patent Office Office européen des brevets

(11) **EP 4 571 769 B1**

(12) **EUROPEAN PATENT SPECIFICATION**

(45) Date of publication and mention of the grant of the patent:
**22.07.2026 Bulletin 2026/30**

(21) Application number: **24820521.3**

(22) Date of filing: **30.08.2024**

(51) International Patent Classification (IPC):
*G16H 20/30* (2018.01)    *G06V 20/52* (2022.01)
*G16H 40/20* (2018.01)    *G16H 40/60* (2018.01)
*G06Q 10/047* (2023.01)    *G06V 40/10* (2022.01)
*G08B 25/01* (2006.01)    *G16H 80/00* (2018.01)
*G08B 27/00* (2006.01)    *H04W 4/90* (2018.01)
*G06Q 10/0631* (2023.01)    *H04W 4/02* (2018.01)
*A61N 1/39* (2006.01)

(52) Cooperative Patent Classification (CPC):
**G16H 40/20; G06Q 10/047; G06Q 10/06311;
G06V 20/53; G06V 40/103; G08B 25/016;
G08B 27/001; G16H 20/30; G16H 40/60;
G16H 80/00; H04W 4/02; H04W 4/90;** A61N 1/3904;
A61N 1/3993

(86) International application number:
**PCT/CN2024/116053**

(87) International publication number:
**WO 2025/086889 (01.05.2025 Gazette 2025/18)**

(54) **INTELLIGENT METHOD AND SYSTEM FOR QUICKLY GETTING AED TO EMERGENCY SCENE, DEVICE, AND MEDIUM**

VERFAHREN UND SYSTEM ZUR INTELLIGENTEN UND SCHNELLEN ERFASSUNG VON AED ZUR ANKUNFT AN EINER NOTFALLUMGEBUNG, VORRICHTUNG UND MEDIUM

PROCÉDÉ ET SYSTÈME INTELLIGENTS POUR AMENER RAPIDEMENT UN DAE À UNE SCÈNE D'URGENCE, DISPOSITIF ET SUPPORT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **25.10.2023 CN 202311389063**

(43) Date of publication of application:
**18.06.2025 Bulletin 2025/25**

(73) Proprietors:
• **Primedic (Jiangsu) Medical Science and Technology Co., Ltd.**
**Zhenjiang, Jiangsu 212300 (CN)**
• **Metrax GmbH**
**78628 Rottweil (DE)**

(72) Inventors:
• **JING, Wei**
**Zhenjiang, Jiangsu 212300 (CN)**

• **HUANG, Hua**
**Zhenjiang, Jiangsu 212300 (CN)**
• **CHEN, Yilin**
**Zhenjiang, Jiangsu 212300 (CN)**
• **LIU, Sixing**
**Zhenjiang, Jiangsu 212300 (CN)**
• **WU, Qun**
**Zhenjiang, Jiangsu 212300 (CN)**

(74) Representative: **Huang, Liwei**
**Cäcilienstraße 12**
**40597 Düsseldorf (DE)**

(56) References cited:
WO-A1-2018/069383    WO-A1-2020/055676
CA-A1- 3 154 933    CN-A- 107 517 447
CN-A- 109 767 837    CN-A- 115 602 306
CN-A- 116 030 952    CN-A- 117 116 453
CN-U- 217 088 165    US-B1- 10 565 845

**Description**

The present invention claims priority to Chinese patent application No. 202311389063.6 filed with CNIPA on October 25, 2023, and entitled "Method and System for Intelligently and Rapidly Acquiring AED to Arrive at First-aid Scene, Device and Medium".

**TECHNICAL FIELD**

The present invention relates to the technical field of cardio-pulmonary resuscitation, in particular to a method and system for intelligently and rapidly acquiring an AED to arrive at a first-aid scene, a device and a medium.

**BACKGROUND**

The statements in this section only provide background art information related to the present invention and do not necessarily constitute the prior art.

Early 85% to 90% of patients with cardiac arrest experience ventricular fibrillation, and the most effective method for treating the ventricular fibrillation is early use of an automated external defibrillator (AED) for defibrillation. After the ventricular fibrillation occurs, the most effective way is to defibrillate within 3-5 minutes. Delaying defibrillation by 1 minute reduces a survival rate by 7% to 10%. About 540000 people die from cardiac arrest in China every year, and placing AED first-aid equipment in public places is an important measure to reduce sudden death caused by cardiac arrest. AED external defibrillation is an important step in cardio pulmonary resuscitation (CPR). The person using the AED is referred to as an implementer of the AED and the CPR (hereinafter referred to as the implementer), and the person receiving the AED external defibrillation is referred to as a recipient the AED and the CPR (hereinafter referred to as a patient).

At present, the AED has been widely used in configurations of public places with high pedestrian flows outside of hospitals, for timely rescue of patients due to sudden cardiac arrest. When a first-aid event occurs, it is necessary to acquire the AED as soon as possible to arrive at the scene. The longer the AED arrives, the lower the survival rate of the patients. However, there is still a low efficiency in how to rapidly respond to acquire the AED and bring the AED to the first-aid scene. Most products at present are dedicated to solving the problems of searching for the AED, reasonable laying of the AED, and an AED delivery manner, and no solution to rapidly acquire the AED to arrive at the scene is available. Moreover, most existing AED delivery solutions rely on rescue volunteers equipped with rescue volunteer terminals to cooperate with rescuers to operate. The rescuer or the rescue volunteer needs to first go to a location of a target AED to acquire the AED, and then return to the first-aid scene from the location of the target AED. When a distance between the rescuer or the rescue volunteer and the target AED is too far, the time required for each round will exceed golden time for optimal rescue, which slows down a rescue speed and cannot improve the survival rate of patients. Document WO2020/055676A1 (AVIVE SOLUTIONS INC [US], 19 March 2020) discloses systems and methods for coordinating AEDs and volunteer responders via a connected network, using device status, location, and travel time to select suitable AEDs and dispatch alerts. Document WO2018/069383A1 (KONINKLIJKE PHILIPS NV [NL], 19 April 2018) discloses systems for routing responders and AEDs to cardiac emergencies using incident location, responder availability, and optimal path calculations. Document CA3154933A1 (AVIVE SOLUTIONS INC [US], 27 May 2021) discloses system and methods for device-based and virtual-assistant-enabled activation of AED responder networks, integrating connected devices and emergency services to dispatch responders and guide them to incidents.

In view of this, a solution of how to acquire the AED at top speed to arrive at the scene after the first-aid event occurs, achieve the most efficient response time, and increase the treatment speed and the survival rate of the patients, has become the research topic to be solved in the present invention.

**SUMMARY**

An objective of the present invention is to provide a method and system for intelligently and rapidly acquiring an AED to arrive at a first-aid scene, a device and a medium, so as to acquire the AED at top speed to arrive at the scene after a first-aid event occurs, achieve the most efficient response time, and increase a treatment speed and a survival rate of patients.

To achieve the above objective, a first aspect of the present invention provides a method for intelligently and rapidly acquiring an AED to arrive at a first-aid scene, and the method includes:

acquiring distress call information of a first-aid event and a geographical location of the first-aid scene, and searching for an available AED near the first-aid event;

performing AED search first-range calculation to search for the available AED closest to the first-aid event and lock the available AED, and measuring an optimal path and a to-be-consumed time for a rescuer to take and deliver the AED back and forth, and outputting AED delivery time A;

performing AED search second-range calculation to search for the available AED closest to the first-aid event and lock the available AED, and measuring the optimal path and the to-be-consumed time for a callable volunteer or AED management personnel near the available AED to acquire the AED and then deliver the AED to the first-aid scene, and outputting AED delivery time B;

performing AED search third-range calculation to search for the available AED with pedestrian traffic near the first-aid event, and calculating a final response probability of pedestrians according to pedestrian traffic data and an average pedestrian response probability of the pedestrians near the acquired available AED within a previous period, and measuring the optimal path and the to-be-consumed time for the pedestrian near the available AED to finally respond to acquire the AED and then deliver the AED to the first-aid scene, and outputting AED delivery time C;

outputting, if the final response probability is greater than a response setting index, an arrival solution with a shortest duration among the AED delivery time A, the AED delivery time B, and the AED delivery time C as a fastest AED arrival solution; outputting, if the final response probability is less than the response setting index, the arrival solution with a shortest duration among the AED delivery time A and the AED delivery time B as a fastest AED arrival solution; and

defining a rescuer, a callable volunteer, AED management personnel, or a responding pedestrian corresponding to the fastest AED arrival solution as AED delivery execution personnel, and displaying the optimal path and the to-be-consumed time information corresponding to the fastest AED arrival solution to the AED delivery execution personnel, and acquiring, by the AED delivery execution personnel, the available AED from the available AED corresponding to the fastest AED arrival solution and delivering the AED to the first-aid scene.

A second aspect of the present invention provides a system for intelligently and rapidly acquiring an AED to arrive at a first-aid scene, used in the method described in the first aspect, and the system includes a first-aid mobile terminal, an intelligent AED device, and a first-aid cloud platform;

the first-aid mobile terminal is a device arranged to issue distress call information of a first-aid event and a geographic location of the first-aid scene, and receiving and displaying the optimal path and the to-be-consumed time information corresponding to a fastest AED arrival solution; the first-aid mobile terminal communicates with the first-aid cloud platform in a remote wireless manner;

the intelligent AED device is a device used for external defibrillation, the intelligent AED device includes an alarm module, a video displaying module, and a video collecting module, and the alarm module is configured to at least give an alarm when receiving AED delivery time C as a fastest AED arrival solution, so that a pedestrian near an available AED is able to respond to the distress call information of the first-aid event in a timely manner; the video displaying module is configured to display the optimal path and the to-be-consumed time information corresponding to the fastest AED arrival solution; the video collecting module is configured to collect a video image near an intelligent AED; and

the first-aid cloud platform communicates with the first-aid mobile terminal and the intelligent AED device in a remote wireless manner, and the first-aid cloud platform is configured to perform AED search first-range calculation, AED search second-range calculation, and AED search third-range calculation after receiving the distress call information of the first-aid event issued by the first-aid mobile terminal, output the fastest AED arrival solution, and display the optimal path and the to-be-consumed time information corresponding to the fastest AED arrival solution to AED delivery execution personnel via the first-aid mobile terminal or the intelligent AED device.

A third aspect of the present invention provides an intelligent AED device, used in the method described in the first aspect, and the intelligent AED device includes a main control module, a video collecting module, a power module, a communication module, a storage module, a video displaying module, and an alarm module;

the main control module is configured to perform information centralization, storage, analysis, and decision-making;

the video collecting module is configured to collect a video image near an intelligent AED;

the power module is configured to supply power to other modules;

the storage module is configured to store AED operation information, video image information, and detection information;

the communication module is responsible for communicating with a first-aid cloud platform;

the video displaying module is configured to display the optimal path and the to-be-consumed time information corresponding to a fastest AED arrival solution; and

the alarm module is configured to give an alarm when receiving AED delivery time C as the fastest AED arrival solution.

A fourth aspect of the present invention provides a readable storage medium, the readable storage medium stores a control program thereon, and the control program, when executed by a processor, causes the processor to execute steps of the method for intelligently and rapidly acquiring the AED to arrive at the first-aid scene as described in the first aspect.

The relevant content of the present invention is explained as follows:

1. through the implementation of the above technical solutions in the present invention, by performing the AED search first-range calculation, the AED search second-range calculation, and the AED search third-range calculation after acquiring the distress call information of the first-aid event, the solution for delivering the AED in the shortest time is outputted to the corresponding rescuer, callable volunteer, AED management personnel, or responding pedestrian. Compared with a manner that can only rely on a volunteer and AED management personnel to deliver the AED in an existing AED delivery solution, the present invention specifically includes the pedestrian located near the available AED as the AED delivery execution personnel to acquire the AED and arrive at the first-aid scene. This is to consider the sudden occurrence of cardiac arrest in patients, the irregular geographical location of the first-aid site, and the possibility of longer delivery time for the available AED by the rescuer, callable volunteer, or the AED management personnel who is closer to the available AED. At this time, through the AED search third-range calculation, the final response probability of the pedestrian is calculated according to the pedestrian traffic data and average response probability of the pedestrian within the previous period near the available AED acquired, the optimal path and the to-be-consumed time for the pedestrian near the available AED to finally respond to acquire the AED and deliver the AED to the first-aid scene is measured, and when the final response probability is greater than the response setting index, the outputted arrival solution with the shortest duration among an AED search first-range, an AED search second-range and an AED search third-range is outputted as the fastest AED arrival solution. Due to the fact that the optimal path and the to-be-consumed time for the pedestrian to finally respond to acquire the AED and deliver the AED to the first-aid scene in the AED search third-range is usually shorter than that in the AED search first-range and the AED search second-range, it can provide the most efficient way to rapidly acquire the AED to arrive at the scene in first-aid scenarios, achieve the most efficient response time, and provide an intelligent computing solution for defibrillation of patients with cardiac arrest as early as possible after ventricular fibrillation occurs, thereby increasing the treatment speed and survival rate of the patients. Moreover, with the popularization of the AED and first-aid knowledge, there will be more and more pedestrians who have received the AED knowledge, and the final response probability of the pedestrians will also increase. After adopting the solution of the present invention, a good solution can be provided for the pedestrians to participate in first aid of the cardiac arrest, the pedestrians participate in the first aid of the cardiac arrest accurately, scientifically and effectively, and another assistance is provided for the patients to receive timely treatment.

2. In the first aspect of the technical solution above, the rescuer initiates the one-click rescue on the first-aid mobile terminal, after the first-aid cloud platform acquires the distress call information and the geographical location of the event, the first-aid cloud platform measures the available AED closest to the geographical location of the first-aid scene and the available AED with the pedestrian traffic near the first-aid event, and definition of the available AED is that an AED equipment state is normal; and

the first-aid cloud platform performs the AED search first-range calculation, the AED search second-range calculation and the AED search third-range calculation, and the first-aid cloud platform outputs the fastest AED arrival solution to the AED delivery execution personnel, and also transmits the optimal path and the to-be-consumed time information corresponding to the fastest AED arrival solution, and information of the AED delivery execution personnel to the first-aid mobile terminal.

The issuing of the distress call information and the acquisition of the fastest AED arrival solution by the rescuer on the first-aid mobile terminal can make the rescuer understand the distance and time for the AED to arrive at the first-aid scene in real time, thus the rescuer can better grasp the relevant situation when implementing CPR on site, which provides assistance for adopting the better CPR, thereby increasing the survival rate of the patients.

3. In the first aspect of the technical solution above, for the measurement of the optimal path and the to-be-consumed time in steps of the AED search first-range calculation, the AED search second-range calculation and the AED search third-range calculation, the optimal path is obtained by using the graph search path algorithm;

in the step of the AED search first-range calculation, the AED delivery time A=(optimal departing path+optimal returning path)/average speed;

in the step of the AED search second-range calculation, the AED delivery time B=(the optimal path from the callable volunteer or AED management personnel to the AED+the optimal path from the AED to the first-aid scene)/average speed; and

in the step of the AED search third-range calculation, the AED delivery time C=the optimal path from the AED to the first-aid scene/average speed.

In this way, after acquiring the distress call information of the first-aid event, the fastest AED arrival solution can be calculated at top speed, so that the patients can receive AED defibrillation treatment faster and more timely.

4. In the first aspect of the technical solution above, the optimal path is acquired by using the following steps:

converting a grid path into a weighted graph;
creating a distance matrix between a node and a starting point according to the weighted graph, setting a value of the starting point to be 0, and setting remaining elements to be infinity;
creating a parent node matrix of the node, and initializing the same to be 0, indicating no parent node;
finding a node with a lowest total evaluation cost among nodes that are not traversed completely in the distance matrix, and setting the node as a current node; setting a current node distance to be infinity and marking the same as the node that is traversed completely; determining untraversed nodes among all adjacent nodes of the current node, judging whether a weighted distance of the adjacent nodes is greater than an edge weight from the current node to a next node, and updating, if so, to the current node;
repeating the above steps until the current node is a target point; and
acquiring the optimal path by completing parent node backtracking according to the parent node matrix.

The method for acquiring the optimal path has the advantages of wide applicability, high efficiency, and the ability to quickly find the optimal solution, which can further improve path planning and resource allocation capabilities in the first-aid event.

5. In the first aspect of the technical solution above, after outputting the arrival solution in the AED search third-range as the fastest AED arrival solution, the fastest AED arrival solution is sent to the available AED, and an alarm is given by the available AED; the pedestrian near the available AED rapidly responds to take the AED, and deliver the AED to the first-aid scene according to navigation information of the optimal path on an AED screen; and if the pedestrian near the available AED that gives the alarm does not respond to acquire the AED within time N, the arrival solution within the shortest duration among the AED delivery time A and the AED delivery time B is re-outputted as the fastest AED arrival solution, wherein a sum of the time N plus the AED delivery time C is greater than the AED delivery time A and the AED delivery time B.

Through the implementation of the above method, it is ensured that in a case of no response from the pedestrians after using the AED search third-range as the fastest AED arrival solution, the arrival solution with the shortest duration among the AED delivery time A and the AED delivery time B can be promptly converted and outputted as the fastest AED arrival solution, so as to provide further guarantee for ensuring the delivery of the AED, thereby enabling the patients to receive AED defibrillation treatment and avoiding unexpected situations.

6. In the first aspect of the technical solution above, when the first-aid event occurs, the rescuer initiates one-click distress call on the first-aid mobile terminal, and the first-aid cloud platform acquires a positioning and specific situation of the scene, measures the available AED closest to the geographical location of the first-aid event and locks the available AED, and measures the optimal path and the to-be-consumed time for the rescuer to take and deliver the AED back and forth; and when it is calculated that the AED delivery time A in the AED search first-range is shortest, the first-aid cloud platform sends the geographic location, an equipment state, a delivery manner, a route map, a real-time location, and delivery countdown information of the AED to a mobile terminal of the rescuer, and the rescuer goes to take the AED and arrives at the first-aid scene. Using the solution of the AED search first-range as the fastest AED arrival solution can allow the rescuer to rapidly know the location and route of the available AED, and acquire the available AED at top speed to enable the patients to receive AED defibrillation treatment in the most effective time.

7. In the first aspect of the technical solution above, when the first-aid event occurs, the rescuer initiates one-click distress call on the first-aid mobile terminal, and the first-aid cloud platform acquires the positioning and specific situation of the scene, measures the available AED closest to the geographical location of the first-aid event, and searches for the callable volunteer and AED management personnel near the available AED; when it is calculated that the AED delivery time B in the AED search second-range is shortest, an event request is sent to a mobile terminal of the callable volunteer or the AED management personnel and the available AED is locked and at the same time, the callable volunteer or the AED management personnel receives the first-aid event on the mobile terminal and handles the same; and the optimal path and the to-be-consumed time for the personnel to acquire the AED and deliver the AED to the first-aid scene is measured, the callable volunteer or the AED management personnel goes to take the AED and arrives at the first-aid scene, and the first-aid cloud platform sends the geographic location, the equipment state, the delivery manner, the route map, the real-time location, and at the same time, the delivery countdown information of the AED to the first-aid mobile terminal. Using the solution of the AED search second-range as the fastest AED arrival solution can allow the callable volunteer or the AED management personnel to rapidly know occurrence of the first-aid event, and the location and route of the available AED, and acquire the available AED at top speed to enable the patients to receive AED defibrillation treatment in the most effective time.

8. In the first aspect of the technical solution above, when the first-aid event occurs, the rescuer initiates one-click distress call on the first-aid mobile terminal, and the first-aid cloud platform acquires the positioning and specific situation of the scene, and searches for the available AED with the highest number of pedestrian traffic near the first-aid event; when it is calculated that the AED delivery time C in the AED search third-range is shortest, the available AED gives a sound or light alarm, first-aid event positioning navigation and specific event description information are sent to the available AED, and an event request is presented on an available AED screen; the pedestrian near the available AED, after noticing the available AED alarm, opens an AED outer box to acquire the AED and handles the first-aid event request; an optimal path and a to-be-consumed time for the pedestrian to acquire the AED and deliver the same to the scene after automatic alarm of the AED is measured, the pedestrian navigates to the first-aid scene according to an equipment information prompt, and the first-aid cloud platform sends the geographic location, the equipment state, the delivery manner, the route map, the real-time location, and at the same time, the delivery countdown information of the AED to the first-aid mobile terminal. Using the solution of the AED search third-range as the fastest AED arrival solution can allow the pedestrian to rapidly know occurrence of the first-aid event and the location and route of the available AED, and acquire the available AED at top speed to enable the patients to receive AED defibrillation treatment in the most effective time.

9. In the first aspect of the technical solution above, in the step of performing the AED search third-range calculation, the pedestrian traffic data is acquired by using the following steps:

collecting an image video near the available AED within a previous period at a moment of acquiring the first-aid information;

performing motion extraction on spatial object information collected in the image video to judge the pedestrian flow near the available AED; and

characterizing a density of the pedestrian flow, and taking an acquired pedestrian flow probability density distribution situation as the pedestrian traffic data.

By acquiring the pedestrian traffic data above, the effective pedestrian traffic data near the available AED within the previous period at the moment of acquiring the first-aid information can be acquired, so as to calculate and output the fastest AED arrival solution more rapidly.

10. In the first aspect of the technical solution above, in the step of performing the AED search third-range calculation, a time period of the previous period is set to be within 10-20 minutes before a moment of receiving the first-aid information near the available AED;

the response setting index is set to be 95%;

the image video is captured by a camera on the available AED;

the final response probability=pedestrian traffic data Y*average pedestrian response probability Z; and

when Y*Z>95% and the AED delivery time C is less than the AED delivery time A and the AED delivery time B, the arrival solution of the AED search third-range calculation is outputted as the fastest AED arrival solution.

By adopting the above steps and related parameter settings, it may be ensured that the information collected in the step of AED search third-range calculation is sufficient to calculate the final response probability, and ensured that the acquisition of the final response probability is reasonable and effective. Therefore, when outputting the arrival solution of the AED search third-range calculation as the fastest AED arrival solution, it can be ensured that the pedestrian responds in a timely manner, acquires the AED in a timely manner, and delivers the AED to the first-aid scene in a timely manner.

11. In the first aspect of the technical solution above, in the step of performing the AED search third-range calculation, a process of calculating the final response probability of the pedestrian according to the pedestrian traffic data and an average pedestrian response probability near the available AED within the previous period is as follows:

collecting the image video near the available AED within the previous period at the moment of acquiring the first-aid information;

establishing a staggered local grid near the available AED; and pre-constructing a pedestrian flow density distribution matrix to represent distribution information of the pedestrian flow in the uniform local grid;

performing motion extraction on spatial object information collected in the image video by using an image filtering method to extract a spatial pedestrian flow information;

projecting the extracted spatial pedestrian flow information onto the local grid in a plane coordinate by using an image space projection method, so as to represent a probability density distribution situation of the pedestrian flow on each grid of the local grid by a plane projection characterization, which serves as the pedestrian traffic data; and

calculating the final response probability of AED response by Poisson distribution of the pedestrian flow probability on the local grid in the plane coordinate.

12. In the first aspect of the technical solution above, performing motion extraction on the spatial object information collected in the image video by using the image filtering method uses the following steps:

reconstructing, for the acquired image video near the available AED, a background image without the pedestrian flow according to pixels with slowly changing pixel values on a timeline of the image video, and updating a background model in real time: still maintaining, for the pixels judged to belong to the pedestrian flow in a detection of the pedestrian flow, an original background pixel value and not updating; and updating, for pixels judged as the background, the background model according to the following rules: $\mathbf{B}_{n+1}(i, j) = \alpha\mathbf{B}_n(i, j) + (1 - \alpha)\mathbf{G}(i, j)$;

wherein, $\alpha \in (0,1)$ is an update rate, $\mathbf{B}_n(i, j)$ represents a pixel value of the pixel of the background image, and $\mathbf{G}(i, j)$ represents a pixel value of the pixel in a current frame;

performing, for continuous video images, continuous Gaussian distribution differential extraction on an $n^{th}$ image to filter background noise and obtain a background image B; and

differentiating the image video by using an HIS space of a color image as a basic space for image differentiation, and binarizing the image acquired after differentiation to detect the pedestrian flow, so as to extract the spatial pedestrian flow information.

Through the above implementation of performing the motion extraction on the spatial object information collected in the image video by using the image filtering method, for video images captured by cameras in the same direction, the pixel values of background pixels change relatively slowly, while the pixel values of pixels corresponding to moving targets change relatively rapidly. Therefore, by collecting a sequence of the video images over a period of time, background images without pedestrian flows may be reconstructed according to the pixels with the slowly changing pixel values on the timeline. At the same time, the background model can be made to be adaptive to changes in external light and can further resist background noise caused by slow rotation of the cameras in the same direction, thereby obtaining more accurate pedestrian traffic data.

13. In the first aspect of the technical solution above, for pre-constructing the pedestrian flow density distribution matrix to represent the distribution information of the pedestrian flow in the uniform local grid, the pedestrian flow density distribution matrix $\mathbf{X}$ is pre-constructed,

$$\mathbf{X} = \begin{bmatrix} \mathbf{X}_{11} & \cdots & \mathbf{X}_{1j} \\ \vdots & \ddots & \vdots \\ \mathbf{X}_{i1} & \cdots & \mathbf{X}_{ij} \end{bmatrix}$$

wherein, each element $X_{ij}$ in the matrix X represents a numerical representation of the pedestrian flow density distribution recorded in the local grid;

each calculated image pixel is projected by using a directional projection transformation matrix H , plane projection is performed on the acquired pedestrian flow density, and the directional projection transformation matrix H is:

$$\mathbf{H} = \begin{bmatrix} \cos\theta & -\sin\theta & h_2 \\ \sin\theta & \cos\theta & h_5 \\ 0 & 0 & 1 \end{bmatrix}$$

wherein, $\theta$ is a rotation angle of pixels in a matrix image, and $h_2$ and $h_5$ are the translation amount of the entire image; the image data is uniformly transformed into a plane representation at the same angle by using projection transformation for characterizing the pedestrian flow density distribution, X = H * (G - B);

the probability of each pedestrian responding to the AED is set to be the same and equal to Z, and the final response probability of the pedestrian is calculated by using Poisson distribution: $P(K = k) = \dfrac{e^{-\lambda}\lambda^k}{k!}$, wherein k represents the number of occurrences of events, and $\lambda$ represents a probability response coefficient under the pedestrian flow distribution; and

for the probability response coefficient $\lambda$ under the pedestrian flow distribution, an expectation of the Poisson distribution is directly related to the pedestrian flow density distribution matrix X, that is, Po($\lambda$) = E(X) * Z, and a value is acquired through a lookup table method.

Through the above calculation of the final response probability for the pedestrian, the calculation process is scientific and reasonable, and the acquired information is effectively utilized for rapid measurement, thereby providing assistance for reliability and accuracy of pedestrian response to the AED in the calculation of the AED search third-range, and avoiding a situation where the pedestrian does not respond after using the AED delivery time C of the AED search third-range as the fastest AED arrival solution.

14. In the technical solution of the present invention, the duration requirements for the AED delivery time A, the AED delivery time B, and the AED delivery time C are to be less than 3 minutes. If all of them are greater than 3 minutes, that is, the AED cannot arrive at the first-aid scene within 3 minutes, the system may inform the rescuer that the AED cannot be acquired, please call 120 (or the first-aid cloud platform contacts 120 directly while the first-aid information is issued, and the relevant information is sent to 120 together) immediately, and at the same time, a CPR operation is performed.

Due to the application of the above solution, the present invention has the following advantages and effects compared to the prior art:

1. through the implementation of the technical solutions in the present invention, by performing the AED search first-range calculation, the AED search second-range calculation, and the AED search third-range calculation after acquiring the distress call information of the first-aid event, the solution for delivering the AED in the shortest time is outputted to the corresponding rescuer, callable volunteer, AED management personnel, or responding pedestrian, so that the AED can be delivered to the first-aid scene at top speed.

2. Through the implementation of the technical solutions in the present invention, compared with the manner that can only rely on the volunteer and the AED management personnel to deliver the AED in the existing AED delivery solution, the present invention specifically includes the pedestrian located near the available AED as the AED delivery execution personnel to acquire the AED and arrive at the first-aid scene. This is to consider the sudden occurrence of cardiac arrest in patients, the irregular geographical location of the first-aid site, and the possibility of longer delivery time for the available AED by the rescuer, callable volunteer, or the AED management personnel who is closer to the available AED. At this time, through the AED search third-range calculation, the final response probability of the pedestrian is calculated according to the pedestrian traffic data and average response probability of the pedestrian within the previous period near the available AED acquired, the optimal path and the to-be-consumed time for the pedestrian near the available AED to finally respond to acquire the AED and deliver the AED to the first-aid scene is measured, and when the final response probability is greater than the response setting index, the outputted arrival solution with the shortest duration among an AED search first-range, an AED search second-range and an AED search third-range is outputted as the fastest AED arrival solution. Due to the fact that the optimal path and the to-be-consumed time for the pedestrian to finally respond to acquire the AED and deliver the AED to the first-aid scene in the AED search third-range is usually shorter than that in the AED search first-range and the AED search second-range, it can provide the most efficient way to rapidly acquire the AED to arrive at the scene in first-aid scenarios, achieve the most efficient response time, and provide an intelligent computing solution for defibrillation of patients with cardiac arrest as early as possible after ventricular fibrillation occurs, thereby increasing the treatment speed and survival rate of the patients.

3. In summary, after adopting the solution of the present invention, a good solution can be provided for the pedestrian to participate in first aid of cardiac arrest, and the pedestrian is involved in the first aid of the cardiac arrest accurately, scientifically, and effectively. Moreover, with the promotion and application of the AED, the response possibility of the pedestrian will gradually increase, and another assistance is provided for the patients to receive timely treatment, thereby ensuring life safety and health protection of the patients.

## BRIEF DESCRIPTION OF THE DRAWINGS

Fig. 1 is a schematic flow diagram of a method for intelligently and rapidly acquiring an AED to arrive at a first-aid scene in an embodiment of the present invention;

Fig. 2 is a schematic flow diagram of performing AED search first-range calculation in a method of an embodiment of the present invention;

Fig. 3 is a schematic scenario diagram of performing AED search first-range calculation in a method of an embodiment of the present invention;

Fig. 4 is a schematic flow diagram of performing AED search second-range calculation in a method of an embodiment of the present invention;

Fig. 5 is a schematic scenario diagram of performing AED search second-range calculation in a method of an embodiment of the present invention;

Fig. 6 is a schematic flow diagram of performing AED search third-range calculation in a method of an embodiment of the present invention;

Fig. 7 is a schematic scenario diagram of performing AED search third-range calculation in a method of an embodiment of the present invention;

Fig. 8 is a schematic flow diagram of AED search range merging calculation in a method of an embodiment of the present invention;

Fig. 9 is a schematic diagram of converting a grid path into a weighted graph in a method of an embodiment of the present invention;

Fig. 10 is a schematic diagram of performing plane projection transformation in a method of an embodiment of the present invention;

Fig. 11 is a system block diagram of a system for intelligently and rapidly acquiring an AED to arrive at a first-aid scene in an embodiment of the present invention; and

Fig. 12 is a system block diagram of an intelligent AED device in an embodiment of the present invention.

## DETAILED DESCRIPTION OF THE EMBODIMENTS

In order to make the above purpose, features and advantages of the present invention more obvious and understandable, the specific implementations of the present invention will be described in detail below with reference to the accompanying drawings. In the following description, numerous specific details are set forth to provide a thorough understanding of the present invention.

Terms herein are only intended to describe specific embodiments and are not intended to limit the present invention. The singular forms such as "one", "this", "such", "present", and "the said", as used herein, also include the plural form.

Terms such as "first" and "second" used herein do not specifically refer to order or priority, nor are they intended to limit the present invention. They are only used to distinguish components or operations described in the same technical terms.

Terms "connection" or "positioning" used herein can refer to that two or more components or devices are in direct physical contact with each other or in indirect physical contact with each other, or that two or more components or devices are in operation or motion with each other.

Terms such as "comprising", "including", "having" used herein are all open-ended terms, meaning including but not limited to.

Terms used herein, unless otherwise specified, usually have the ordinary meaning of each term used in this art, in the content of the present invention, and in the special content. Certain terms used to describe the present invention will be discussed below or elsewhere in this specification to provide additional guidance to those skilled in the art regarding the description of the case.

The present invention aims to provide a method and system for intelligently and rapidly acquiring an AED to arrive at a first-aid scene, a device and a medium, so as to acquire the AED at top speed to arrive at the scene after a first-aid event occurs, achieve the most efficient response time, and thus increase a treatment speed and a survival rate of patients. In particular, a good solution can be provided for the pedestrian to participate in first aid of cardiac arrest, and the pedestrian is involved in the first aid of the cardiac arrest accurately, scientifically, and effectively. Moreover, with the promotion and application of the AED, the response possibility of the pedestrian will gradually increase, and another assistance is provided for the patients to receive timely treatment, thereby ensuring life safety and health protection of the patients.

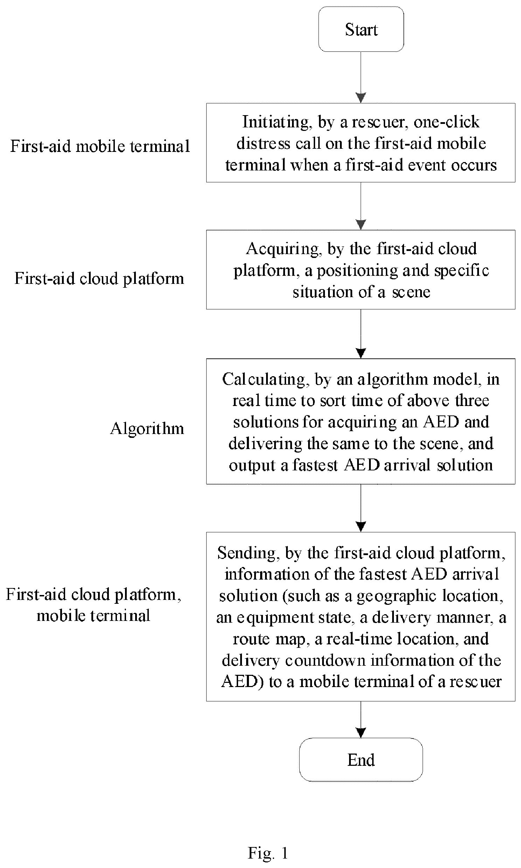

Embodiment 1, as shown in Fig. 1, Embodiment 1 of the present invention provides a method for intelligently and rapidly acquiring an AED to arrive at a first-aid scene, and the method includes:

distress call information of a first-aid event and a geographical location of a first-aid scene are acquired, and an available AED near the first-aid event is searched;

AED search first-range calculation is performed to search for an available AED closest to the first-aid event and lock the available AED, an optimal path and a to-be-consumed time for a rescuer to take and deliver the AED back and forth are measured, and AED delivery time A is outputted;

AED search second-range calculation is performed to search for an available AED closest to the first-aid event and lock the available AED, an optimal path and a to-be-consumed time for a callable volunteer or AED management personnel near the available AED to acquire the AED and then deliver the AED to the first-aid scene are measured, and AED delivery time B is outputted;

AED search third-range calculation is performed to search for an available AED with pedestrian traffic near the first-aid event, a final response probability of pedestrians is calculated according to pedestrian traffic data and an average pedestrian response probability of the pedestrians near the acquired available AED within a previous period, an optimal path and a to-be-consumed time for the pedestrian near the available AED to finally respond to acquire the AED and then deliver the AED to the first-aid scene are measured, and AED delivery time C is outputted;

if the final response probability is greater than a response setting index, an arrival solution with a shortest duration among the AED delivery time A, the AED delivery time B, and the AED delivery time C is outputted as a fastest AED arrival solution; if the final response probability is less than the response setting index, an arrival solution with a shortest duration among the AED delivery time A and the AED delivery time B is outputted as a fastest AED arrival solution; and

a rescuer, a callable volunteer, AED management personnel, or a responding pedestrian corresponding to the fastest AED arrival solution is defined as AED delivery execution personnel, optimal path and to-be-consumed time information corresponding to the fastest AED arrival solution is displayed to the AED delivery execution personnel, and the delivery execution personnel acquires the available AED from the available AED corresponding to the fastest AED arrival solution and delivers the AED to the first-aid scene.

In Embodiment 1 of the present invention, the rescuer initiates one-click rescue on a first-aid mobile terminal, after a first-aid cloud platform acquires the distress call information and the geographical location of the event, the first-aid cloud platform measures the available AED closest to the geographical location of the first-aid scene and the available AED with the pedestrian traffic near the first-aid event, and definition of the available AED is that an AED equipment state is normal; and the first-aid cloud platform performs the AED search first-range calculation, the AED search second-range calculation and the AED search third-range calculation, and the first-aid cloud platform outputs the fastest AED arrival solution to the AED delivery execution personnel, and also transmits the optimal path and the to-be-consumed time information corresponding to the fastest AED arrival solution, and information of the AED delivery execution personnel to the first-aid mobile terminal. The issuing of the distress call information and the acquisition of the fastest AED arrival solution by the rescuer on the first-aid mobile terminal can make the rescuer understand a distance and time for the AED to arrive at the first-aid scene in real time, thus the rescuer can better grasp the relevant situation when implementing CPR on site, which provides assistance for adopting the better CPR, thereby increasing a survival rate of the patients.

In Embodiment 1 of the present invention, when the first-aid event occurs, the rescuer initiates one-click distress call on the first-aid mobile terminal, and the first-aid cloud platform acquires a positioning and specific situation of the scene, measures the available AED closest to the geographical location of the first-aid event and locks the available AED, and measures the optimal path and the to-be-consumed time for the rescuer to take and deliver the AED back and forth; and when it is calculated that the AED delivery time A in an AED search first-range is shortest, the first-aid cloud platform sends the geographic location, an equipment state, a delivery manner, a route map, a real-time location, and delivery countdown information of the AED to a mobile terminal of the rescuer, and the rescuer goes to take the AED and arrives at the first-aid scene. Using the solution of the AED search first-range as the fastest AED arrival solution can allow the rescuer to rapidly know the location and route of the available AED, and acquire the available AED at top speed to enable the patient to receive AED defibrillation treatment in the most effective time.

In Embodiment 1 of the present invention, when the first-aid event occurs, the rescuer initiates one-click distress call on the first-aid mobile terminal, and the first-aid cloud platform acquires a positioning and specific situation of the scene, measures the available AED closest to the geographical location of the first-aid event, and searches for the callable volunteer and AED management personnel near the available AED; when it is calculated that the AED delivery time B in an AED search second-range is shortest, an event request is sent to a mobile terminal of the callable volunteer or the AED management personnel and the available AED is locked, and at the same time, the callable volunteer or the AED management personnel receives the first-aid event on the mobile terminal and handles the same; and the optimal path and the to-be-consumed time for the personnel to acquire the AED and deliver the AED to the first-aid scene is measured, the callable volunteer or the AED management personnel goes to take the AED and arrives at the first-aid scene, and the first-aid cloud platform sends the geographic location, the equipment state, the delivery manner, the route map, the real-time location, and at the same time, the delivery countdown information of the AED to the first-aid mobile terminal. Using the solution of the AED search second-range as the fastest AED arrival solution can allow the callable volunteer or the AED management personnel to rapidly know occurrence of the first-aid event, and the location and route of the available AED, and acquire the available AED at top speed to enable the patient to receive AED defibrillation treatment in the most effective time.

In Embodiment 1 of the present invention, when the first-aid event occurs, the rescuer initiates one-click distress call on the first-aid mobile terminal, and the first-aid cloud platform acquires a positioning and specific situation of the scene, and searches for the available AED with the highest number of pedestrian traffic near the first-aid event; when it is calculated that the AED delivery time C in an AED search third-range is shortest, the available AED gives a sound or light alarm, first-aid event positioning navigation and specific event description information are sent to the available AED, and an event request is presented on an available AED screen; the pedestrian near the available AED, after noticing the available AED alarm, opens an AED outer box to acquire the AED and handles the first-aid event request; an optimal path and a to-be-consumed time for the pedestrian to acquire the AED and deliver the same to the scene after automatic alarm of the AED is measured, the pedestrian navigates to the first-aid scene according to an equipment information prompt, and the first-aid cloud platform sends the geographic location, the equipment state, the delivery manner, the route map, the real-time location, and at the same time, the delivery countdown information of the AED to the first-aid mobile terminal. Using the solution of the AED search third-range as the fastest AED arrival solution can allow the pedestrian to rapidly know occurrence of the first-aid event and the location and route of the available AED, and acquire the available AED at top speed to enable the patient to receive AED defibrillation treatment in the most effective time.

In Embodiment 1 of the present invention, in the step of performing the AED search third-range calculation, the pedestrian traffic data is acquired by using the following steps: an image video near the available AED within a previous period at a moment of acquiring the first-aid information is collected; motion extraction is performed on spatial object information collected in the image video to judge the pedestrian flow near the available AED; and a density of the pedestrian flow is characterized, and an acquired pedestrian flow probability density distribution situation is taken as the pedestrian traffic data. By acquiring the pedestrian traffic data above, the effective pedestrian traffic data near the available AED within the previous period at the moment of acquiring the first-aid information can be acquired, so as to calculate and output the fastest AED arrival solution more rapidly.

In Embodiment 1 of the present invention, in the step of performing the AED search third-range calculation, a time period of the previous period is set to be within 10-20 minutes before a moment of receiving the first-aid information near the available AED; the response setting index is set to be 95%; the image video is captured by a camera on the available AED; the final response probability=the pedestrian traffic data Y*average pedestrian response probability Z; and when Y*Z>95% and the AED delivery time C is less than the AED delivery time A and the AED delivery time B, the arrival solution of the AED search third-range calculation is outputted as the fastest AED arrival solution. By adopting the above steps and related parameter settings, it may be ensured that the information collected in the step of the AED search third-range calculation is sufficient to calculate the final response probability, and ensured that the acquisition of the final response probability is reasonable and effective. Therefore, when outputting the arrival solution of the AED search third-range calculation as the fastest AED arrival solution, it can be ensured that the pedestrian responds in a timely manner, acquires the AED in a timely manner, and delivers the AED to the first-aid scene in a timely manner.

Below, Embodiment 1 of the present invention is further illustrated by a more detailed embodiment.

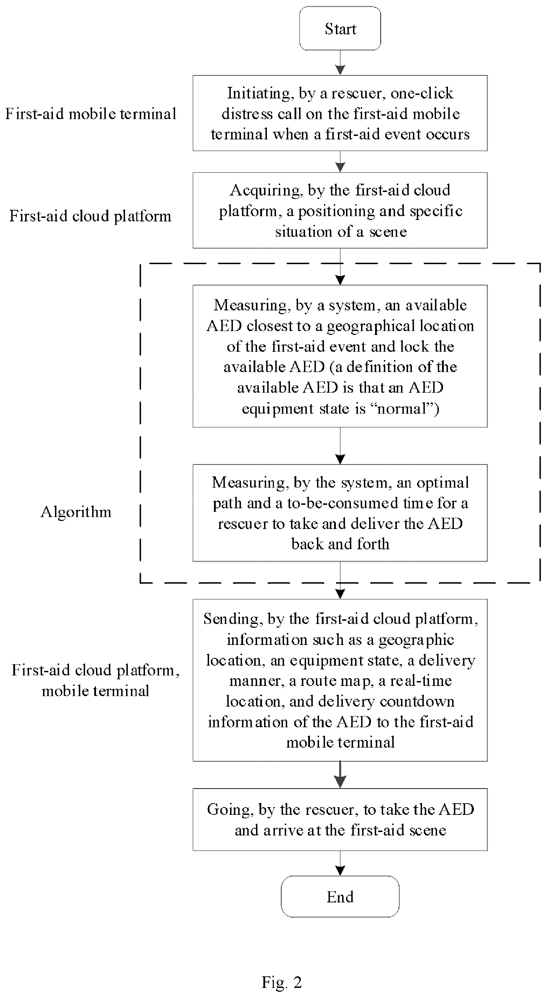

The following detailed contents included in the method for intelligently and rapidly acquiring the AED to arrive at the first-aid scene in this embodiment may refer to Fig. 2 to Fig. 5.

The embodiment of the present invention mainly aims to provide an intelligent computing solution for rapidly acquiring the AED to arrive at the scene, and an intelligent AED device itself may receive the first-aid information sent by the cloud platform and give an alarm. The pedestrian near the intelligent AED device rapidly responds to take the AED and deliver the AED to the first-aid scene according to navigation on an AED screen. By combining the AED camera to acquire the nearby pedestrian flow density and the average pedestrian response probability of the AED in the past 15 minutes of the intelligent AED device, the to-be-consumed time of the solution is calculated. Only when the pedestrian response probability of in the solution meets a condition and the total to-be-consumed time is less than time required for the rescuer or volunteer to take and deliver the AED back and forth, the solution can be established and pushed for execution by the system. If the solution is executed, it will be the most efficient way to acquire the AED in the first-aid scenario.

When the first-aid event occurs, the rescuer declares the first-aid event on the mobile terminal and initiates a request to search for the AED. After receiving the event request, the first-aid cloud platform starts to calculate the optimal solution for acquiring the AED in real time, namely, the fastest AED arrival solution. The definition of the fastest AED arrival solution is the fastest way for the AED to arrive at the first-aid scene. A backend feeds the fastest way back to the mobile terminal of the rescuer and informs a user in real-time of the distance and time for the AED to arrive at the scene.

The specific solutions of the embodiment of the present invention are as follows.

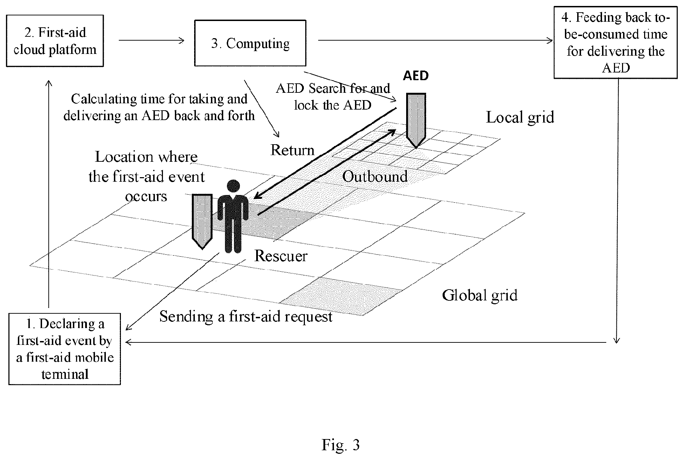

A flow diagram for the AED search first-range calculation may refer to Fig. 2, and a schematic scenario diagram may refer to Fig. 3. The AED search first-range calculation is as follows:

(1) the rescuer declares the first-aid event on the first-aid mobile terminal and sends the positioning and specific situation of the scene to the first-aid cloud platform;

(2) the first-aid cloud platform processes the event after receiving the first-aid event and searches for the nearest available AED;

(3) the first-aid cloud platform measures the available AED closest to the geographical location of the first-aid event and locks the available AED (the definition of the available AED is that an AED equipment state is "normal");

(4) the first-aid cloud platform measures the optimal path and the to-be-consumed time for the rescuer to take and deliver the AED back and forth (such as measuring the time according to an average speed of 2m/s for a normal adult);

(5) a calculation formula A:

(optimal departing path+optimal returning path)/average speed=the AED delivery time A;

the definition of the optimal path is: the shortest to-be-consumed time;

the definition of the average speed is: an average speed of reasonable transportation modes such as walking and running that are suitable for the current scenario;

(6) the first-aid cloud platform sends the geographic location, the equipment state, the delivery manner, the route map, the real-time location, and the delivery countdown information of the AED to the mobile terminal of the rescuer; and

(7) the rescuer goes to take the AED and arrives at the first-aid scene.

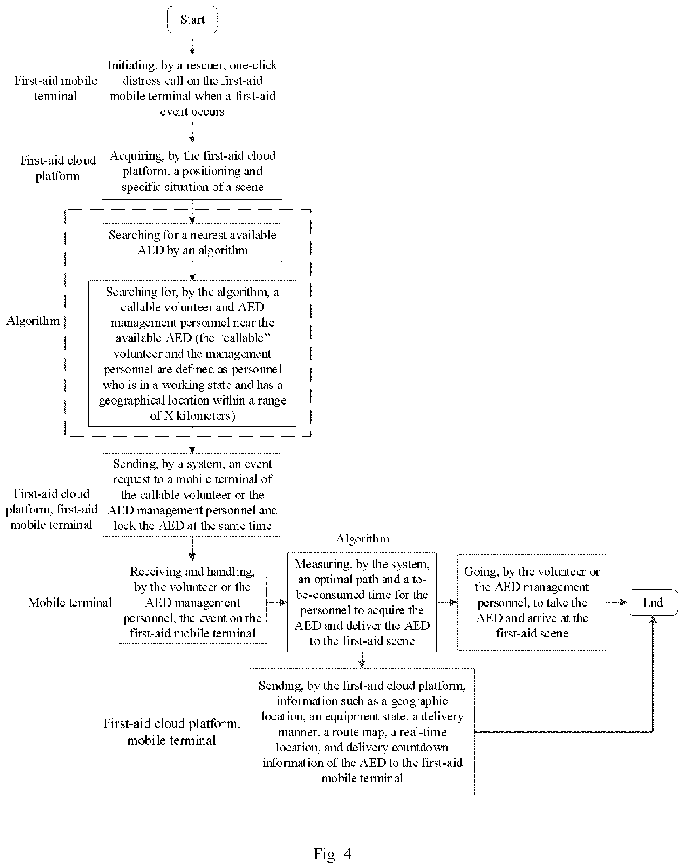

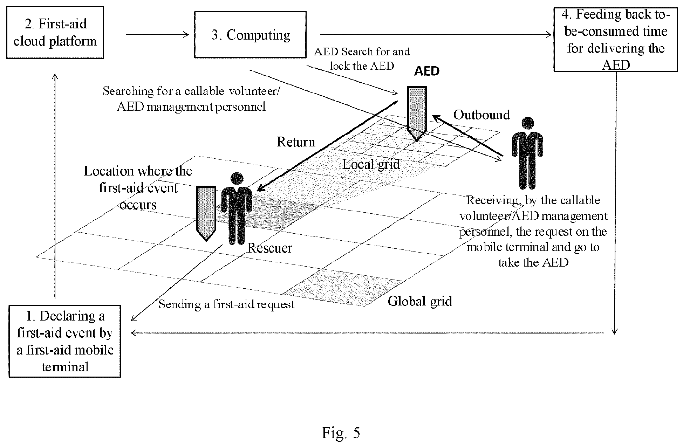

A flow diagram for the AED search second-range calculation may refer to Fig. 4, and a schematic scenario diagram may refer to Fig. 5. The AED search second-range calculation is as follows:

(1) the rescuer declares the first-aid event on the first-aid mobile terminal and sends the positioning and specific situation of the scene to the first-aid cloud platform;
(2) the first-aid cloud platform processes the event after receiving the first-aid event, searches for the nearest available AED, and searches for the callable volunteer and AED management personnel near the available AED (the "callable" volunteer and the management personnel are defined as personnel who is in a working state and has a geographical location within a range of X kilometers);
(3) the first-aid cloud platform sends the event request to a mobile terminal of the callable volunteer or the AED management personnel and at the same time, locks the AED;
(4) the callable volunteer or the AED management personnel receives and handles the first-aid event on the mobile terminal;
(5) the first-aid cloud platform measures the optimal path and the to-be-consumed time for the personnel to acquire the AED and deliver the AED to the first-aid scene;
(6) a calculation formula B:
(an optimal path distance from the callable volunteer or the AED management personnel to the AED+an optimal path distance from the AED to the scene)/average speed=the AED delivery time B;
(7) the first-aid cloud platform sends the geographic location, the equipment state, the delivery manner, the route map, the real-time location, and the delivery countdown information of the AED to the first-aid mobile terminal; and
(8) the callable volunteer or the AED management personnel goes to take the AED and arrives at the first-aid scene.

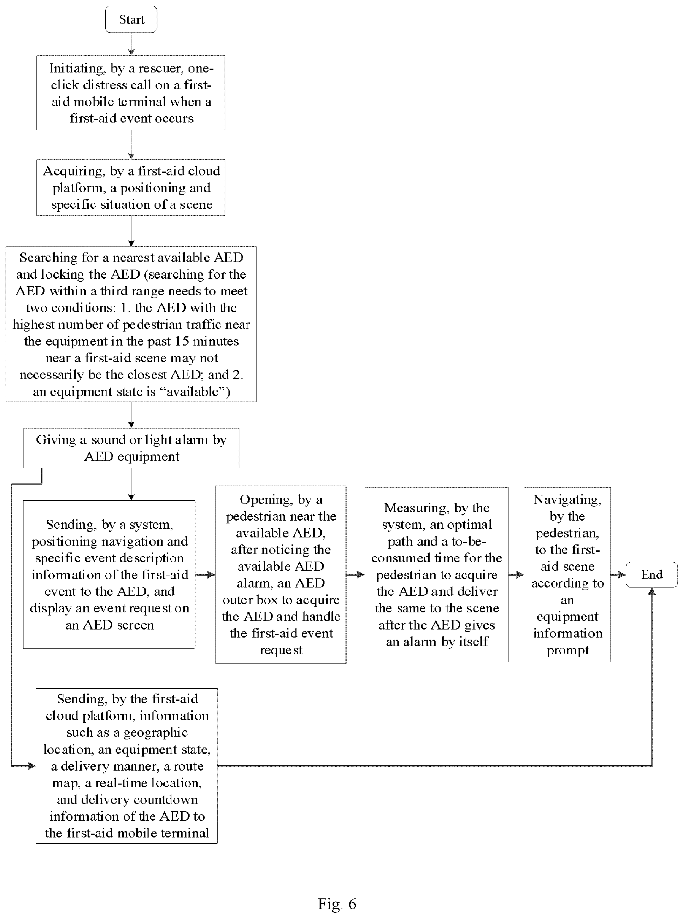

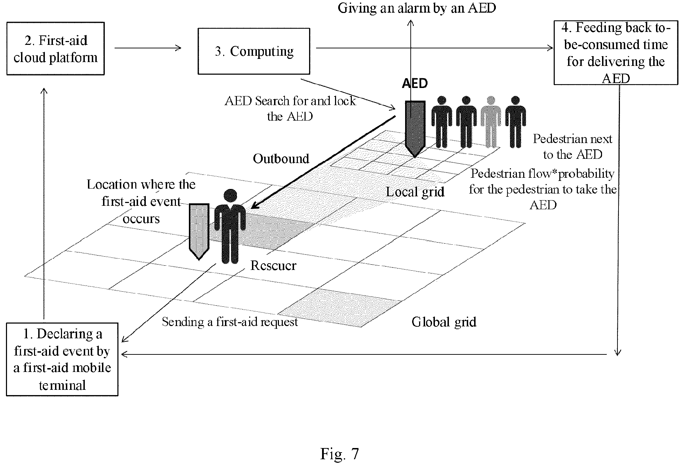

A flow diagram for the AED search third-range calculation may refer to Fig. 6, and a schematic scenario diagram may refer to Fig. 7. The AED search third-range calculation is as follows:

(1) the rescuer declares the first-aid event on the first-aid mobile terminal and sends the positioning and specific situation of the scene to the first-aid cloud platform;
(2) the first-aid cloud platform processes the event after receiving the first-aid event, searches for the available AED in the first-aid scene, and locks the AED (searching for the AED within the third range needs to meet two conditions: 1. the AED with the highest number of pedestrian traffic near the equipment in the past 15 minutes near the first-aid scene may not necessarily be the closest AED; and 2. the equipment state is "available");
(3) the intelligent AED device gives a sound prompt word "first-aid event has occurred" and a red light alarm;
(4) the first-aid cloud platform sends positioning navigation and specific event description information of the first-aid event to the AED, and the AED screen displays the event request;
(5) the first-aid cloud platform measures the optimal path and the to-be-consumed time for the pedestrian to acquire the AED and deliver the same to the scene after the AED alarms by itself;
(6) a calculation formula C:

calculation of the final response probability of the pedestrian: the pedestrian traffic data Y at the geographic location where the AED is located on the past 15 minutes captured by the AED camera*average pedestrian response probability Z;
calculation of the time for the pedestrian to acquire the AED and deliver the same to the scene: the optimal path distance from the AED to the scene/average speed =AED delivery time C; (because in step (2), the equipment may not necessarily be the same AED within the first and second ranges, the AED delivery time C may not necessarily be less than the AED delivery time A and the AED delivery time B);
when Y*Z>95% and the AED delivery time C is less than the AED delivery time A and the AED delivery time B, the system provides feedback that the fastest AED arrival solution of the first-aid mobile terminal of the rescuer is the AED search third-range calculation solution;

(7) the pedestrian near the available AED, after noticing the available AED alarm, opens an AED outer box to acquire the AED and handles the first-aid event request;
(8) the first-aid cloud platform sends the geographic location, the equipment state, the delivery manner, the route map, the real-time location, and the delivery countdown information of the AED to the first-aid mobile terminal; and
(9) the pedestrian navigates to the first-aid scene according to an equipment information prompt.

From the three schematic diagrams shown in Fig. 3, Fig. 5, and Fig. 7, it can be known that in the AED search third-range calculation solution, if the locked available AED is the same as the AED in the first and second ranges, the total travel distance by the pedestrian to acquire the AED and deliver the same to the scene is shortest. Therefore, theoretically, the time C is shortest under normal circumstances. But if the AED locked in the third range is different from that in the first and second ranges, and the pedestrian response speed is related to the pedestrian flow density near the AED acquired by the AED camera in the past 15 minutes and an average probability of acquiring the AED, only when the total to-be-consumed time of the solution is less than the to-be-consumed time of the first and second ranges, the solution may be established and pushed for execution by the system. If the third solution is pushed to the user as a feasible solution, but the AED is still not acquired by the pedestrian within N seconds, the system recommends the second ranked solution as the final feasible solution. The above computing process is continuously processed in real time by the first-aid cloud platform to ensure that the AED arrives at the scene with the fastest solution within 3 minutes.

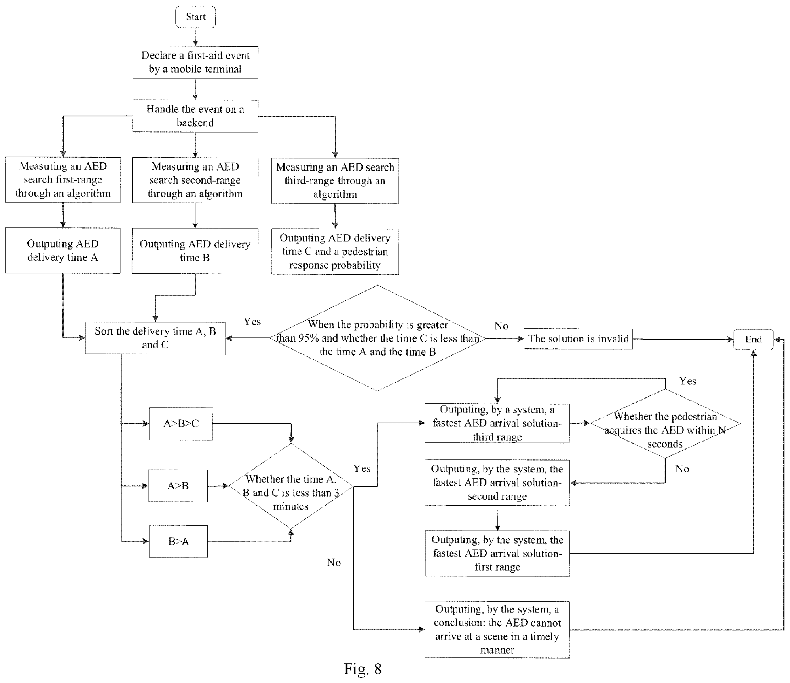

Based on this, an algorithm model of the first-aid cloud platform calculates in real time to sort the time of the above three measuring manners for acquiring the AED and delivering the same to the scene, and finally outputs the fastest solution result to be sent to the mobile terminal of the rescuer. A schematic flow diagram for merging calculation of the AED search range may refer to Fig. 8. The rescuer may see the AED delivery manner, the route map and the real-time location, and delivery countdown on the mobile terminal. If the AED can arrive at the scene within 3 minutes, the mobile terminal will display the fastest acquisition solution. If the AED cannot arrive at the scene within 3 minutes, the system informs the rescuer that the AED cannot be acquired, please call 120 immediately, and at the same time, a CPR operation is performed (or the first-aid cloud platform contacts 120 directly while the first-aid information is issued, and the relevant information is sent to 120 together).

For acquiring the optimal path in the above methods, please refer to the following.

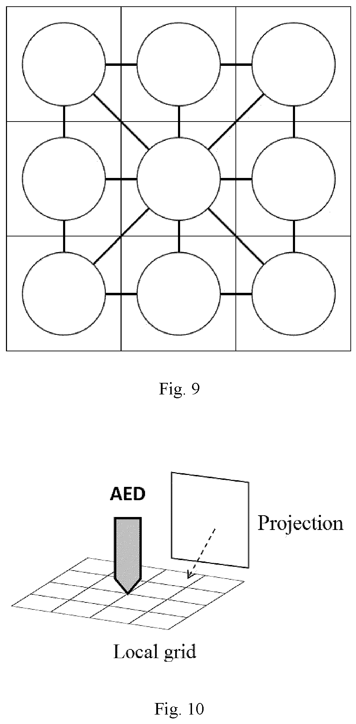

(1) first, a grid path is converted into a weighted graph, as shown in Fig. 9;
(2) a distance matrix between a node and a starting point is created, a value of the starting point is set to be 0, and remaining elements are set to be infinity (inf);
(3) a parent node matrix of the node is created, and is initialized to be 0, indicating no parent node;
(4) during each iteration update, a node (the node with a smallest edge weighted sum) with a lowest total evaluation cost among nodes that are not traversed completely is found in the distance matrix, and set as a current node; to prevent further traversal, a current node distance is set to be infinity and marked as the node that is traversed completely; untraversed nodes among all adjacent nodes of the current node are determined, whether a weighted distance of the adjacent nodes is greater than an edge weight from the current node to a next node is judged, and if so, it is updated to the current node;
(5) step (4) is repeated until the current node is a target point; and
(6) the optimal path is completed by backtracking the parent node according to the parent node matrix.

For the calculation of the final response probability in the AED search third-range calculation, please refer to the following.

the estimation of the pedestrian flow density in the environment is reflected through video image information collected by a video collecting module (camera) on the intelligent AED device, and the local grid is established to record the pedestrian flow density distribution. Finally, the response probability of the pedestrian flow to the AED alarm is estimated through the Poisson distribution. The main process is:

(1) a staggered local grid near the available AED is first established. A pedestrian flow density distribution matrix is pre-constructed to represent distribution information of the pedestrian flow in the uniform local grid.
(2) Then, motion extraction is performed on spatial object information collected in the image video by using an image filtering method so as to judge the estimation of the surrounding pedestrian flow.
(3) Next, extracted spatial pedestrian flow information is projected onto plane coordinate grid cells by using an image space projection method.
(4) Finally, the final response probability of AED response is calculated by Poisson distribution of the pedestrian flow probability on the local grid in the plane.

The pedestrian flow density distribution matrix X is used to represent the distribution of the pedestrian flow in a unit grid;

$$\mathbf{X} = \begin{bmatrix} \mathbf{X}_{11} & \cdots & \mathbf{X}_{1j} \\ \vdots & \ddots & \vdots \\ \mathbf{X}_{i1} & \cdots & \mathbf{X}_{ij} \end{bmatrix} \qquad (1)$$

wherein, each element $X_{ij}$ in the matrix X represents a numerical representation of the pedestrian flow density distribution recorded in the unit grid.

For video images captured by cameras in the same direction, pixel values of background pixels change relatively slowly, while pixel values of pixels corresponding to moving targets change relatively rapidly. Therefore, by collecting a sequence of the video images over a period of time, background images without pedestrian flows may be reconstructed according to the pixels with the slowly changing pixel values on a timeline. At the same time, to make the background model adaptive to changes in external light, the background model needs to be updated in real time. The following update method may be used: in the detection of the pedestrian flow, for the pixels judged to belong to the pedestrian flow, the original background pixel values are still maintained and not updated. For the pixels judged as the background, the background model is updated according to the following rules:

$$B_{t+1}(i,j) = \alpha B_t(i,j) + (1-\alpha)G(i,j) \qquad (2)$$

wherein, $\alpha \in (0,1)$ is an update rate, $B_t(i,j)$ represents a pixel value of the pixel of the background image at a moment t, and $G(i,j)$ represents a pixel value of the pixel in a current frame.

The background subtraction operation utilizes an HIS space of color images as a basic space for image differentiation according to human visual characteristics. In a high saturation (high S) region, chromaticity information represented by H is more obvious, and an H value may be used for differentiation; and in a low saturation (low S) region, brightness information represented by is more obvious, and an I value may be used for differentiation. By binarizing the image acquired after differentiation, the pedestrian flow can be detected.

At the same time, in order to resist background noise caused by slow rotation of the cameras in the same direction, the background noise is further filtered. For continuous video images, continuous Gaussian distribution differential extraction is performed on an $n^{th}$ image at the moment t;

$$g_{n,t} = (1-\alpha)g_{n,t-1} + \alpha G_{n,t} \qquad (3)$$

wherein, $g_{n,t}$ is the Gaussian distribution of the $n^{th}$ image at the time t, which is specifically:

$$\mu_t = (1-\rho)\mu_{t-1} + \rho G_t$$
$$\sigma_t^2 = (1-\rho)\sigma_{t-1}^2 + \rho(G_t - \mu_t)^T(G_t - \mu_t) \qquad (4)$$
$$\rho = \alpha\eta(X_t|\mu_t, \sigma_t^2)$$

wherein, $\mu_t$ represents a mean value of n image pixels at the moment t, $\sigma_t^2$ represents a variance of the n image pixels at the moment t, $\rho$ represents the Gaussian distribution probability density estimated using binary distribution, and $\eta$ represents a binary distribution function.

By optimizing noise through Gaussian, the background image B is:

$$B = \underset{b}{\arg\min} \left( \sum_{k=1}^{b} g_{n,t} > W \right) \qquad (5)$$

In the formula, W is a minimum weight of the background noise, generally $1 \leq W \leq n$, and in practical situations, this weight is related to a slow rotation rate of the camera.

In order to project the spatial pedestrian flow density information represented in the video onto the plane coordinate of the geographic location for representation, the spatial characterization information of the video image captured by the camera needs to be subjected to plane projection transformation as shown in Fig. 10.

Each calculated image pixel is projected by using a directional projection transformation matrix H , plane projection is performed on the acquired pedestrian flow density, and the projection matrix is:

$$H = \begin{bmatrix} \cos\theta & -\sin\theta & h_2 \\ \sin\theta & \cos\theta & h_5 \\ 0 & 0 & 1 \end{bmatrix} \qquad (6)$$

In the formula, $\theta$ is a rotation angle of pixels in a matrix image, and $h_2$ and $h_5$ are the translation amount of the entire image.

Through the above transformation, the image data is uniformly transformed into a plane representation at the same angle by using projection transformation for characterizing the pedestrian flow density distribution, according to the formula (1), each element acquired from matrix X is the pedestrian flow distribution situation in the unit grid. Specifically, the background pixels B are corrected by subtracting the formulas (2) -(5) from the pixels G for image acquisition, and then calculation is performed by the projection through the affine transformation of the formula (6), as shown in the following formula:

$$X = H * (G - B) \qquad (7)$$

The plane projection representation may represent the probability density distribution situation of the pedestrian flow on each grid. Assuming that each person has the same probability of responding to the AED and the probability is equal to Z, the response probability of the pedestrian flow is calculated by using Poisson distribution, as follows:

$$P(K = k) = \frac{e^{-\lambda}\lambda^k}{k!} * Z \qquad (8)$$

In the above formula, k represents the number of occurrences of the event, and the pedestrian flow response needing the AED in the solution is at least once. Therefore, for the case, the above formula takes k = 1 ; $\lambda$ represents the probability response coefficient under the pedestrian flow distribution. In a case of assuming that each person's response probability to the AED is a fixed value, an expected probability is related to the density of the pedestrian flow. Therefore, the np expectation of Poisson distribution may be directly related to the mean value of the pedestrian flow density distribution matrix, as follows:

$$Po(\lambda) = E(X) \qquad (9)$$

A value of $\lambda$ may be acquired by using a lookup table method.

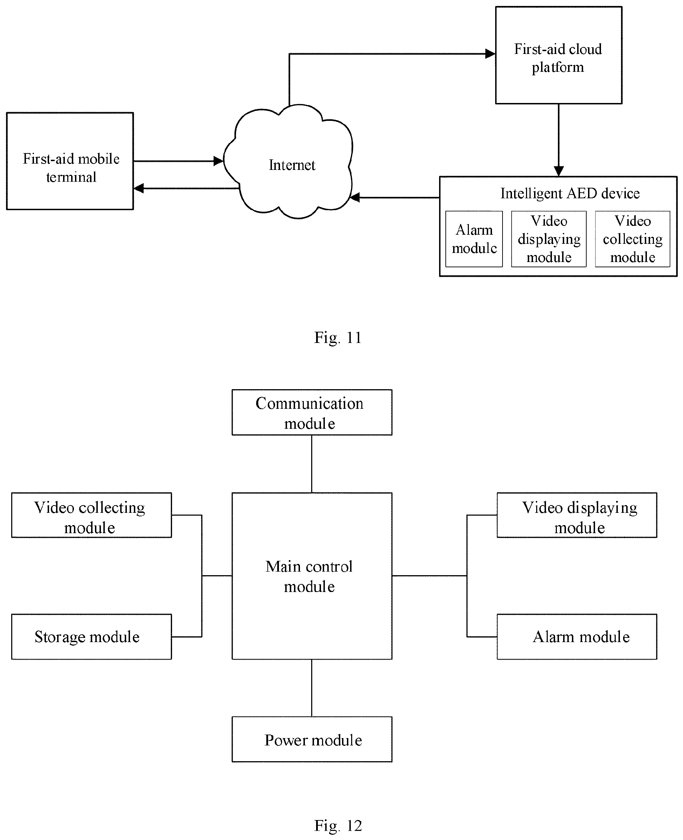

Embodiment 2, as shown in Fig. 11, Embodiment 2 of the present invention provides a system for intelligently and rapidly acquiring an AED to arrive at a first-aid scene, and the system includes a first-aid mobile terminal, an intelligent AED device, and a first-aid cloud platform; wherein

the first-aid mobile terminal is a device arranged to issue distress call information of a first-aid event and a geographic location of a first-aid scene, and receiving and displaying the optimal path and the to-be-consumed time information corresponding to a fastest AED arrival solution; the first-aid mobile terminal communicates with the first-aid cloud platform in a remote wireless manner;

the intelligent AED device is a device used for external defibrillation, the intelligent AED device includes an alarm module, a video displaying module, and a video collecting module, and the alarm module is configured to at least give an alarm when receiving AED delivery time C as a fastest AED arrival solution, so that a pedestrian near an available AED is able to respond to the distress call information of the first-aid event in a timely manner; the video displaying module is configured to display the optimal path and the to-be-consumed time information corresponding to the fastest AED arrival solution; and the video collecting module is configured to collect a video image near the intelligent AED; and

the first-aid cloud platform communicates with the first-aid mobile terminal and the intelligent AED device in a remote wireless manner, and the first-aid cloud platform is configured to perform AED search first-range calculation, AED search second-range calculation, and AED search third-range calculation after receiving the distress call information of the first-aid event issued by the first-aid mobile terminal, output the fastest AED arrival solution, and display the optimal path and the to-be-consumed time information corresponding to the fastest AED arrival solution to AED delivery execution personnel via the first-aid mobile terminal or the intelligent AED device.

In Embodiment 2 of the present invention, the first-aid mobile terminal may be a mobile terminal, such as a smartphone and a tablet computer, with a real-time network communication function, and a user may initiate a first-aid request. The first-aid cloud platform is a dispatch center of the first-aid cloud platform, which acquires the first-aid request and pushes an optimal AED rapid acquisition solution to the mobile terminal. An intelligent AED communicates with a first-aid cloud platform server through a remote wireless manner (including but not limited to a cellular network, and Wi-Fi). The first-aid mobile terminal communicates with the first-aid cloud platform server through a remote wireless manner (including but not limited to a cellular network, and Wi-Fi).

Specifically,

1. the AED is connected to the first-aid cloud platform server through the Internet, and the platform acquires a geographic location where the AED is located and an equipment operation situation, and matches a distance between the event and the available intelligent AED device according to a location of the first-aid request event;

2. the first-aid cloud platform server receives the first-aid event request from the first-aid mobile terminal, computes and outputs a fastest AED delivery response solution, outputs a result, and sends the result to a mobile terminal of the rescuer; and

3. the first-aid cloud platform is connected to mobile terminals of a volunteer and the intelligent AED device management personnel through the Internet, and locks current available personnel according to a work state, service region, and a geographic location of the personnel.

Embodiment 3, as shown in Fig. 12, Embodiment 3 of the present invention provides an intelligent AED device, used in the method described in Embodiment 1 of the present invention, and the intelligent AED device includes a main control module, a video collecting module, a power module, a communication module, a storage module, a video displaying module, and an alarm module;

the main control module is configured to perform information centralization, storage, analysis, and decision-making; the video collecting module is configured to collect a video image near an intelligent AED; the power module is configured to supply power to other modules; the storage module is configured to store AED operation information, video image information, and detection information; the communication module is responsible for communicating with a first-aid cloud platform; the video displaying module is configured to display optimal path and the to-be-consumed time information corresponding to a fastest AED arrival solution; and the alarm module is configured to give an alarm when receiving AED delivery time C as the fastest AED arrival solution.

Embodiment 4, Embodiment 4 of the present invention further provides a readable storage medium, the readable storage medium stores a control program thereon, and the control program, when executed by a processor, causes the processor to execute steps of the method for intelligently and rapidly acquiring the AED to arrive at the first-aid scene as described in Embodiment 1 of the present invention.

The implementation of the various embodiments above has achieved the objective of the present invention, by performing the AED search first-range calculation, the AED search second-range calculation, and the AED search third-range calculation after acquiring the distress call information of the first-aid event, the solution for delivering the AED in the shortest time is outputted to the corresponding rescuer, callable volunteer, AED management personnel, or responding pedestrian. Compared with a manner that can only rely on a volunteer and AED management personnel to deliver the AED in an existing AED delivery solution, the present invention specifically includes the pedestrian located near the available AED as the AED delivery execution personnel to acquire the AED and arrive at the first-aid scene. This is to consider the sudden occurrence of cardiac arrest in patients, the irregular geographical location of the first-aid site, and the possibility of longer delivery time for the available AED by the rescuer, callable volunteer, or the AED management personnel who is closer to the available AED. At this time, through the AED search third-range calculation, the final response probability of the pedestrian is calculated according to the pedestrian traffic data and average response probability of the pedestrian within the previous period near the available AED acquired, the optimal path and the to-be-consumed time for the pedestrian near the available AED to finally respond to acquire the AED and deliver the AED to the first-aid scene is measured, and when the final response probability is greater than the response setting index, the outputted arrival solution with the shortest duration among an AED search first-range, an AED search second-range and an AED search third-range is outputted as the fastest AED arrival solution. Due to the fact that the optimal path and the to-be-consumed time for the pedestrian to finally respond to acquire the AED and deliver the AED to the first-aid scene in the AED search third-range is usually shorter than that in the AED search first-range and the AED search second-range, it can provide the most efficient way to rapidly acquire the AED to arrive at the scene in first-aid scenarios, achieve the most efficient response time, and provide an intelligent computing solution for defibrillation of patients with cardiac arrest as early as possible after ventricular fibrillation occurs, thereby increasing the treatment speed and survival rate of the patients. Moreover, with the popularization of the AED and first-aid knowledge, there will be more and more pedestrians who have received the AED knowledge, and the final response probability of the pedestrians will also increase. After adopting the solution of the present invention, a good solution can be provided for the pedestrians to participate in first aid of the cardiac arrest, the pedestrians participate in the first aid of the cardiac arrest accurately, scientifically and effectively, and another assistance is provided for the patients to receive timely treatment.

The above embodiments are only intended to illustrate the technical concept and characteristics of the present invention, and the objective thereof is to enable those skilled in the art to understand and implement the content of the present invention, and cannot limit the scope of protection of the present invention.

**Claims**

1. A method for intelligently and rapidly acquiring an AED for arriving at a first-aid scene, comprising:

acquiring distress call information of a first-aid event and a geographical location of the first-aid scene, and searching for an available AED near the first-aid event;

performing AED search first-range calculation to search for the available AED closest to the first-aid event and locking this available AED, and calculating an optimal path and a to-be-consumed time for a rescuer to go forth to take the AED and deliver the AED back, and outputting AED delivery time A;

performing AED search second-range calculation to search for the available AED closest to the first-aid event, and calculating an optimal path and a to-be-consumed time for a callable volunteer or AED management personnel near the available AED to acquire the AED and then deliver the AED to the first-aid scene, and outputting AED delivery time B;

performing AED search third-range calculation to search for the available AED with pedestrian traffic near the first-aid event, and calculating a final response probability of pedestrians according to acquired pedestrian traffic data and an average pedestrian response probability of the pedestrians near the available AED within a previous period, and calculating an optimal path and a to-be-consumed time for the pedestrian near the available AED to finally respond to acquire the AED and then deliver the AED to the first-aid scene, and outputting AED delivery time C;

if the final response probability is greater than a response setting index, outputting an arrival solution with a shortest duration among the AED delivery time A, the AED delivery time B, and the AED delivery time C as a fastest AED arrival solution;

if the final response probability is less than the response setting index, outputting an arrival solution with a shortest duration among the AED delivery time A and the AED delivery time B as a fastest AED arrival solution; and

defining a rescuer, a callable volunteer, AED management personnel, or a responding pedestrian corresponding to the fastest AED arrival solution as AED delivery execution personnel, and displaying the optimal path and the to-be-consumed time information corresponding to the fastest AED arrival solution to the AED delivery execution personnel, and acquiring, by the AED delivery execution personnel, the available AED from the location of the available AED corresponding to the fastest AED arrival solution and delivering the AED to the first-aid scene.

2. The method for intelligently and rapidly acquiring the AED to arrive at the first-aid scene according to claim 1, wherein

the rescuer initiates one-click rescue on a first-aid mobile terminal, after a first-aid cloud platform acquires the distress call information and the geographical location of the event, the first-aid cloud platform calculates the available AED closest to the geographical location of the first-aid scene and the available AED with the pedestrian traffic near the first-aid event, wherein the available AED is an AED equipment in normal state; and

the first-aid cloud platform performs the AED search first-range calculation, the AED search second-range calculation and the AED search third-range calculation, and the first-aid cloud platform outputs the fastest AED arrival solution to the AED delivery execution personnel, and also transmits the optimal path and the to-be-consumed time information corresponding to the fastest AED arrival solution, and information of the AED delivery execution personnel to the first-aid mobile terminal.

3. The method for intelligently and rapidly acquiring the AED to arrive at the first-aid scene according to claim 1, wherein

for calculation of the optimal path and the to-be-consumed time of the AED search first-range calculation, the AED search second-range calculation and the AED search third-range calculation, the optimal path is acquired by using a graph search path algorithm;

in performing the AED search first-range calculation, the AED delivery time A = ( optimal departing path + optimal returning path ) / average speed;

in performing the AED search second-range calculation, the AED delivery time B = ( the optimal path from the callable volunteer or AED management personnel to the AED + the optimal path from the AED to the first-aid scene ) / average speed; and

in performing the AED search third-range calculation, the AED delivery time C = the optimal path from the AED to the first-aid scene / average speed.

4. The method for intelligently and rapidly acquiring the AED to arrive at the first-aid scene according to claim 3, wherein the optimal path is acquired by using following steps:

converting a grid path into a weighted graph;

creating a distance matrix between a node and a starting point according to the weighted graph, setting a value of the starting point to be 0, and setting remaining elements to be infinity;

creating a parent node matrix of the node, wherein initialization is 0, indicating no parent node;

finding a node with a lowest total evaluation cost among nodes that have not been completely traversed in the distance matrix, and setting the node as a current node; setting the current node to have a infinity distance and marking the current node as completely traversed; determining untraversed nodes among all adjacent nodes of the current node, judging whether a weighted distance of an adjacent node is greater than an edge weight from the current node to a next node, and if so, updating the adjacent node to be the current node;

repeating the above steps until the current node is a target point; and

acquiring the optimal path by completing parent node backtracking according to the parent node matrix.

5. The method for intelligently and rapidly acquiring the AED to arrive at the first-aid scene according to claim 1, wherein after outputting the arrival solution in an AED search third-range as the fastest AED arrival solution, the fastest AED arrival solution is sent to the available AED, and an alarm is given by the available AED; the pedestrian near the available AED rapidly responds to take the AED, and delivers the AED to the first-aid scene according to navigation information of the optimal path on an AED screen; and if the pedestrian near the available AED giving the alarm does not respond to acquire the AED within time N, the arrival solution within the shortest duration among the AED delivery time A and the AED delivery time B is outputted as the fastest AED arrival solution, wherein a sum of the time N plus the AED delivery time C is greater than the AED delivery time A and is greater than the AED delivery time B.

6. The method for intelligently and rapidly acquiring the AED to arrive at the first-aid scene according to claim 1, wherein when the first-aid event occurs, the rescuer initiates one-click distress call on a first-aid mobile terminal, and a first-aid cloud platform acquires a positioning and specific situation of the scene, calculates the available AED closest to the geographical location of the first-aid event and locks the available AED, and calculates the optimal path and the to-be-consumed time for the rescuer to take and deliver the AED back and forth; and when it is calculated that the AED delivery time A in an AED search first-range is shortest, the first-aid cloud platform sends a geographic location of the AED, an equipment state, a delivery manner, a route map, a real-time location, and delivery countdown information to a mobile terminal of the rescuer, and the rescuer goes to take the AED and arrives at the first-aid scene.

7. The method for intelligently and rapidly acquiring the AED to arrive at the first-aid scene according to claim 1, wherein when the first-aid event occurs, the rescuer initiates one-click distress call on a first-aid mobile terminal, and a first-aid cloud platform acquires a positioning and specific situation of the scene, calculates the available AED closest to the geographical location of the first-aid event, and searches for the callable volunteer and AED management personnel near the available AED; when it is calculated that the AED delivery time B in an AED search second-range is shortest, an event request is sent to a mobile terminal of the callable volunteer or the AED management personnel and the available AED is locked at the same time, and the callable volunteer or the AED management personnel receives the first-aid event request on the mobile terminal and handles the first-aid event request; and the optimal path and the to-be-consumed time for the personnel to acquire the AED and deliver the AED to the first-aid scene are calculated, the callable volunteer or the AED management personnel goes to take the AED and arrives at the first-aid scene, and at the same time, the first-aid cloud platform sends the geographic location of the AED, an equipment state, a delivery manner, a route map, a real-time location, and delivery countdown information to the first-aid mobile terminal.

8. The method for intelligently and rapidly acquiring the AED to arrive at the first-aid scene according to claim 1, wherein when the first-aid event occurs, the rescuer initiates one-click distress call on a first-aid mobile terminal, and a first-aid cloud platform acquires a positioning and specific situation of the scene, and searches for the available AED with the highest number of pedestrian traffic near the first-aid event; when it is calculated that the AED delivery time C in an AED search third-range is shortest, the available AED gives a sound or light alarm, a first-aid event positioning navigation and a specific event description information are sent to the available AED, and an event request is presented on an available AED screen; the pedestrian near the available AED, after noticing the available AED alarm, opens an AED outer box to acquire the AED and handles the first-aid event request; the optimal path and the to-be-consumed time for the pedestrian to acquire the AED and deliver the AED to the scene after automatic alarm of the AED are calculated, the pedestrian navigates to the first-aid scene according to an equipment information prompt, and at the same time, the first-aid cloud platform sends the geographic location of the AED, an equipment state, a delivery manner, a route map, a real-time location, and delivery countdown information to the first-aid mobile terminal.

9. The method for intelligently and rapidly acquiring the AED to arrive at the first-aid scene according to claim 1, wherein in performing the AED search third-range calculation, the pedestrian traffic data are acquired by using following steps:

collecting an image video near the available AED within a previous period before a moment of receiving the first-aid information;

performing motion extraction on spatial object information collected in the image video to judge the pedestrian flow near the available AED; and

characterizing a density of the pedestrian flow, and taking an acquired pedestrian flow probability density distribution situation as the pedestrian traffic data.

10. The method for intelligently and rapidly acquiring the AED to arrive at the first-aid scene according to claim 9, wherein

in performing the AED search third-range calculation, a time period of the previous period is set to be within 10-20 minutes before the moment of receiving the first-aid information near the available AED;

the response setting index is set to be 95%;

the image video is captured by a camera on the available AED;

the final response probability=the pedestrian traffic data Y*average pedestrian response probability Z; and

when Y*Z>95% and the AED delivery time C is less than the AED delivery time A and the AED delivery time B, the arrival solution of the AED search third-range calculation is outputted as the fastest AED arrival solution.

11. The method for intelligently and rapidly acquiring the AED to arrive at the first-aid scene according to claim 1, wherein in performing the AED search third-range calculation, a process of calculating the final response probability of the pedestrian according to the pedestrian traffic data and an average pedestrian response probability near the available AED within the previous period is as follows:

collecting an image video near the available AED within a previous period before a moment of receiving the first-aid information;

establishing a staggered local grid near the available AED; and pre-constructing a pedestrian flow density distribution matrix to represent a distribution information of the pedestrian flow in a uniform local grid;

performing motion extraction on spatial object information collected in the image video by using an image filtering method to extract a spatial pedestrian flow information;

projecting the extracted spatial pedestrian flow information onto the local grid in a plane coordinate by using an image space projection method, to represent a probability density distribution situation of the pedestrian flow on each grid of the local grid by a plane projection characterization, which serves as the pedestrian traffic data; and calculating the final response probability of AED response by Poisson distribution of the pedestrian flow probability on the local grid in the plane coordinate.

12. The method for intelligently and rapidly acquiring the AED to arrive at the first-aid scene according to claim 11, wherein the step of performing motion extraction on the spatial object information collected in the image video by using the image filtering method uses following steps:

reconstructing, for the acquired image video near the available AED, a background image without the pedestrian flow according to pixels with slowly changing pixel values on a timeline of the image video, and updating a background model in real time; for the pixels judged as belonging to the pedestrian flow in a detection of the pedestrian flow, maintaining an original background pixel value and not updating; and for pixels judged as the background, updating the background model according to the following rules: $B_{n+1}(i, j) = \alpha B_n(i, j) + (1 - \alpha)G(i, j)$; wherein, $\alpha \in (0,1)$ is an update rate, $B_n(i, j)$ represents a pixel value of the pixel of the background image, and $G(i, j)$ represents a pixel value of the pixel in a current frame;

performing, for continuous video images, continuous Gaussian distribution differential extraction on an $n^{th}$ image to filter background noise and acquire a background image B; and

differentiating the image video by using an HIS space of a color image as a basic space for image differentiation, and binarizing the image acquired after differentiation to detect the pedestrian flow, to extract the spatial pedestrian flow information.

13. An intelligent AED device, for use in the method according to any one of claims 1 to 10, wherein

the intelligent AED device comprises a main control module, a video collecting module, a power module, a communication module, a storage module, a video displaying module, and an alarm module;

the main control module is configured to perform information centralization, storage, analysis, and decision-making;

the video collecting module is configured to collect a video image near an intelligent AED;

the power module is configured to supply power to other modules;

the storage module is configured to store AED operation information, video image information, and detection information;

the communication module is responsible for communicating with a first-aid cloud platform;

the video displaying module is configured to display the optimal path and the to-be-consumed time information corresponding to a fastest AED arrival solution; and

the alarm module is configured to give an alarm when receiving AED delivery time C as the fastest AED arrival solution.

14. A system for intelligently and rapidly acquiring an AED to arrive at a first-aid scene, for use in the method according to any one of claims 1 to 10, wherein

the system comprises a first-aid mobile terminal, the intelligent AED device according to claim 13, and a first-aid cloud platform;

the first-aid mobile terminal is a device configured to send distress call information of a first-aid event and a geographic location of the first-aid scene, and to receive and display the optimal path and the to-be-consumed time information corresponding to a fastest AED arrival solution; the first-aid mobile terminal communicates with the first-aid cloud platform in a remote wireless manner;

the intelligent AED device is a device used for external defibrillation; and

the first-aid cloud platform communicates with the first-aid mobile terminal and the intelligent AED device in a remote wireless manner, and the first-aid cloud platform is configured to perform AED search first-range calculation, AED search second-range calculation, and AED search third-range calculation after receiving the distress call information of the first-aid event sent by the first-aid mobile terminal, output the fastest AED arrival solution, and display the optimal path and the to-be-consumed time information corresponding to the fastest AED arrival solution to an AED delivery execution personnel via the first-aid mobile terminal or the intelligent AED device.

15. A readable storage medium, wherein

the readable storage medium stores a control program thereon, and the control program, when executed by a processor, causes the processor to execute the method according to any one of claims 1 to 10.

**Patentansprüche**

1. Verfahren zum intelligenten und rapiden Erwerb eines AED, um an einer Erste-Hilfe-Umgebung anzukommen, umfassend:

Erwerben von Notrufinformationen eines Erste-Hilfe-Ereignisses und eines geografischen Standortes der Erste-Hilfe-Umgebung und Suchen nach einem verfügbaren AED nahe dem Erste-Hilfe-Ereignis;

Durchführen von Erstbereichsberechnung der AED-Suche, um nach dem verfügbaren AED zu suchen, der dem Erste-Hilfe-Ereignis am nächsten ist, und Sperren dieses verfügbaren AED und Berechnen eines optimalen Weges und einer zu verbrauchenden Zeit für einen Retter, um vorwärts zu gehen, um den AED zu nehmen und den AED zurückzuliefern, und Ausgeben von AED-Lieferzeit A;

Durchführen von Zweitbereichsberechnung der AED-Suche, um nach dem verfügbaren AED zu suchen, der dem Erste-Hilfe-Ereignis am nächsten ist, und Berechnen eines optimalen Weges und einer zu verbrauchenden Zeit für einen anrufbaren Freiwilligen oder AED-Managementpersonal nahe dem verfügbaren AED, um den AED zu erwerben und dann den AED an die Erste-Hilfe-Umgebung zu liefern, und Ausgeben von AED-Lieferzeit B;

Durchführen von Drittbereichsberechnung der AED-Suche, um nach dem verfügbaren AED mit Fußgängerverkehr nahe dem Erste-Hilfe-Ereignis zu suchen, und Berechnen einer finalen Reaktionswahrscheinlichkeit von Fußgängern gemäß erworbenen Fußgängerverkehrsdaten und einer durchschnittlichen Fußgängerreaktionswahrscheinlichkeit der Fußgänger nahe dem verfügbaren AED innerhalb eines vorherigen Zeitraums und Berechnen eines optimalen Weges und einer zu verbrauchenden Zeit für den Fußgänger nahe dem verfügbaren AED, um schließlich zu reagieren, um den AED zu erwerben und dann den AED an die Erste-Hilfe-Umgebung zu liefern, und Ausgeben von AED-Lieferzeit C;

falls die finale Reaktionswahrscheinlichkeit größer als ein Reaktionsfestlegungsindex ist, Ausgeben einer Ankunftslösung mit einer kürzesten Dauer unter der AED-Lieferzeit A, der AED-Lieferzeit B und der AED-Lieferzeit C als eine zügigste AED-Ankunftslösung;

falls die finale Reaktionswahrscheinlichkeit weniger als der Reaktionsfestlegungsindex ist, Ausgeben einer Ankunftslösung mit einer kürzesten Dauer unter der AED-Lieferzeit A und der AED-Lieferzeit B als eine zügigste AED-Ankunftslösung; und

Definieren eines Retters, eines anrufbaren Freiwilligen, von AED-Managementpersonal oder eines reagierenden Fußgängers entsprechend der zügigsten AED-Ankunftslösung als AED-Lieferausführungspersonal und Anzeigen des optimalen Weges und der zu verbrauchenden Zeitinformationen entsprechend der zügigsten AED-Ankunftslösung für das AED-Lieferausführungspersonal und Erwerben, durch das AED-Lieferausführungspersonal, des verfügbaren AED von dem Standort des verfügbaren AED entsprechend der zügigsten AED-Ankunftslösung und Liefern des AED an die Erste-Hilfe-Umgebung.

2. Verfahren zum intelligenten und rapiden Erwerb des AED, um an der Erste-Hilfe-Umgebung anzukommen, gemäß Anspruch 1, wobei

der Retter Ein-Klick-Rettung an einem mobilen Erste-Hilfe-Endgerät initiiert, nachdem eine Erste-Hilfe-Cloud-Plattform die Notrufinformationen und den geografischen Standort des Ereignisses erwirbt, die Erste-Hilfe-Cloud-Plattform den verfügbaren AED, der dem geografischen Standort der Erste-Hilfe-Umgebung am nächsten ist, und den verfügbaren AED mit dem Fußgängerverkehr nahe dem Erste-Hilfe-Ereignis berechnet, wobei der verfügbare AED eine AED-Ausrüstung im Normalzustand ist; und

die Erste-Hilfe-Cloud-Plattform die Erstbereichsberechnung der AED-Suche, die Zweitbereichsberechnung der AED-Suche und die Drittbereichsberechnung der AED-Suche durchführt und die Erste-Hilfe-Cloud-Plattform die zügigste AED-Ankunftslösung an das AED-Lieferausführungspersonal ausgibt und auch den optimalen Weg und die zu verbrauchenden Zeitinformationen entsprechend der zügigsten AED-Ankunftslösung und Informationen des AED-Lieferausführungspersonals an das mobile Erste-Hilfe-Endgerät überträgt.

3. Verfahren zum intelligenten und rapiden Erwerb des AED, um an der Erste-Hilfe-Umgebung anzukommen, gemäß Anspruch 1, wobei

zur Berechnung des optimalen Weges und der zu verbrauchenden Zeit der Erstbereichsberechnung der AED-Suche, der Zweitbereichsberechnung der AED-Suche und der Drittbereichsberechnung der AED-Suche der optimale Weg durch Verwenden eines Graphensuchwegalgorithmus erworben wird;

beim Durchführen der Erstbereichsberechnung der AED-Suche die AED-Lieferzeit A = (optimaler Abfahrtsweg + optimaler Rückfahrweg) / durchschnittliche Geschwindigkeit;

beim Durchführen der Zweitbereichsberechnung der AED-Suche die AED-Lieferzeit B = (der optimale Weg von dem anrufbaren Freiwilligen oder AED-Managementpersonal zu dem AED + der optimale Weg von dem AED zu der Erste-Hilfe-Umgebung) / durchschnittliche Geschwindigkeit; und

beim Durchführen der Drittbereichsberechnung der AED-Suche die AED-Lieferzeit C = der optimale Weg von dem AED zu der Erste-Hilfe-Umgebung / durchschnittliche Geschwindigkeit.

4. Verfahren zum intelligenten und rapiden Erwerb des AED, um an der Erste-Hilfe-Umgebung anzukommen, gemäß Anspruch 3, wobei
der optimale Weg durch Verwenden folgender Schritte erworben wird:

Umwandeln eines Gitterweges in einen gewichteten Graphen;
Erstellen einer Abstandsmatrix zwischen einem Knoten und einem Startpunkt gemäß dem gewichteten Graphen, Festlegen eines Wertes des Startpunktes auf 0, und Festlegen von übrigen Elementen auf unendlich;
Erstellen einer übergeordneten Knotenmatrix des Knotens, wobei Initialisierung 0 ist, was keinen übergeordneten Knoten angibt;
Finden eines Knotens mit niedrigsten Gesamtbewertungskosten unter Knoten, die in der Abstandsmatrix nicht vollständig durchlaufen worden sind, und Festlegen des Knotens als einen aktuellen Knoten; Festlegen des aktuellen Knotens, sodass er einen Unendlichkeitsabstand aufweist, und Markieren des aktuellen Knotens als vollständig durchlaufen; Bestimmen von nicht durchlaufenen Knoten unter allen benachbarten Knoten des aktuellen Knotens, Beurteilen, ob ein gewichteter Abstand eines benachbarten Knotens größer als ein Kantengewicht von dem aktuellen Knoten zu einem nächsten Knoten ist, und falls ja, Aktualisieren des benachbarten Knotens, um der aktuelle Knoten zu sein;
Wiederholen der obigen Schritte, bis der aktuelle Knoten ein Zielpunkt ist; und
Erwerben des optimalen Weges durch Abschließen von Rückverfolgung des übergeordneten Knotens gemäß der Matrix des übergeordneten Knotens.

5. Verfahren zum intelligenten und rapiden Erwerb des AED, um an der Erste-Hilfe-Umgebung anzukommen, gemäß Anspruch 1, wobei

nach dem Ausgeben der Ankunftslösung in einer AED-Drittbereichssuche als die zügigste AED-Ankunftslösung, die zügigste AED-Ankunftslösung an den verfügbaren AED gesendet wird und ein Alarm durch den verfügbaren AED gegeben wird; der Fußgänger nahe dem verfügbaren AED rapide reagiert, um den AED zu nehmen, und den AED an die Erste-Hilfe-Umgebung gemäß Navigationsinformationen des optimalen Weges auf einem AED-Bildschirm liefert; und falls der Fußgänger nahe dem verfügbaren AED, der den Alarm gibt, nicht reagiert, um den AED innerhalb von Zeit N zu erwerben, die Ankunftslösung innerhalb der kürzesten Dauer unter der AED-Lieferzeit A und der AED-Lieferzeit B als die zügigste AED-Ankunftslösung ausgegeben wird, wobei eine Summe aus der Zeit N plus der AED-Lieferzeit C größer als die AED-Lieferzeit A ist und größer als die AED-Lieferzeit B ist.

6. Verfahren zum intelligenten und rapiden Erwerb des AED, um an der Erste-Hilfe-Umgebung anzukommen, gemäß Anspruch 1, wobei

wenn das Erste-Hilfe-Ereignis eintritt, der Retter Ein-Klick-Notruf an einem mobilen Erste-Hilfe-Endgerät initiiert und eine Erste-Hilfe-Cloud-Plattform eine Positionierung und spezifische Situation der Umgebung erwirbt, den verfügbaren AED, der dem geografischen Standort des Erste-Hilfe-Ereignisses am nächsten ist, berechnet und den verfügbaren AED sperrt, und den optimalen Weg und die zu verbrauchende Zeit für den Retter berechnet, um den AED hin und her zu nehmen und zu liefern; und wenn berechnet wird, dass die AED-Lieferzeit A in einer AED-Erstbereichssuche am kürzesten ist, die Erste-Hilfe-Cloud-Plattform einen geografischen Standort des AED, einen Ausrüstungszustand, eine Lieferweise, eine Wegkarte, einen Echtzeitstandort und Liefercountdowninformationen an ein mobiles Endgerät des Retters sendet, und der Retter geht, um den AED zu nehmen und an der Erste-Hilfe-Umgebung ankommt.

7. Verfahren zum intelligenten und rapiden Erwerb des AED, um an der Erste-Hilfe-Umgebung anzukommen, gemäß Anspruch 1, wobei

wenn das Erste-Hilfe-Ereignis eintritt, der Retter Ein-Klick-Notruf an einem mobilen Erste-Hilfe-Endgerät initiiert und eine Erste-Hilfe-Cloud-Plattform eine Positionierung und spezifische Situation der Umgebung erwirbt, den verfügbaren AED berechnet, der dem geografischen Standort des Erste-Hilfe-Ereignisses am nächsten ist, und nach dem anrufbaren Freiwilligen und AED-Managementpersonal nahe dem verfügbaren AED sucht; wenn berechnet wird, dass die AED-Lieferzeit B in einer AED-Zweitbereichssuche am kürzesten ist, eine Ereignisanfrage an ein mobiles Endgerät des anrufbaren Freiwilligen oder des AED-Managementpersonals gesendet wird und der verfügbare AED zu der gleichen Zeit gesperrt wird und der anrufbare Freiwillige oder das AED-Managementpersonal die Erste-Hilfe-Ereignisanfrage an dem mobilen Endgerät empfängt und die Erste-Hilfe-Ereignisanfrage bearbeitet; und der optimale Weg und die zu verbrauchende Zeit für das Personal, um den AED zu erwerben und den AED an die Erste-Hilfe-Umgebung zu liefern, berechnet werden, der anrufbare Freiwillige oder das AED-Managementpersonal geht, um den AED zu nehmen und an der Erste-Hilfe-Umgebung ankommt, und zu der gleichen Zeit die Erste-Hilfe-Cloud-Plattform den geografischen Standort des AED, einen Ausrüstungszustand, eine Lieferweise, eine Wegkarte, einen Echtzeitstandort und Liefercountdowninformationen an das mobile Erste-Hilfe-Endgerät sendet.

8. Verfahren zum intelligenten und rapiden Erwerb des AED, um an der Erste-Hilfe-Umgebung anzukommen, gemäß Anspruch 1, wobei

wenn das Erste-Hilfe-Ereignis eintritt, der Retter Ein-Klick-Notruf an einem mobilen Erste-Hilfe-Endgerät initiiert und eine Erste-Hilfe-Cloud-Plattform eine Positionierung und spezifische Situation der Umgebung erwirbt und nach dem verfügbaren AED mit der höchsten Anzahl an Fußgängerverkehr nahe dem Erste-Hilfe-Ereignis sucht; wenn berechnet wird, dass die AED-Lieferzeit C in einer AED-Drittbereichssuche am kürzesten ist, der verfügbare AED einen Ton- oder Lichtalarm gibt, eine Erste-Hilfe-Ereignis-Positionierungsnavigation und eine spezifische Ereignisbeschreibungsinformation an den verfügbaren AED gesendet werden und eine Ereignisanfrage auf einem verfügbaren AED-Bildschirm präsentiert wird; der Fußgänger nahe dem verfügbaren AED, nach dem Bemerken des verfügbaren AED-Alarms, eine AED-Außenbox öffnet, um den AED zu erwerben, und die Erste-Hilfe-Ereignisanfrage bearbeitet; der optimale Weg und die zu verbrauchende Zeit für den Fußgänger, um den AED zu erwerben und den AED an die Umgebung nach automatischem Alarm des AED zu liefern, berechnet werden, der Fußgänger zu der Erste-Hilfe-Umgebung gemäß einer Ausrüstungsinformationsaufforderung navigiert und zu der gleichen Zeit die Erste-Hilfe-Cloud-Plattform den geografischen Standort des AED, einen Ausrüstungszustand, eine Lieferweise, eine Wegkarte, einen Echtzeitstandort und Liefercountdowninformationen an das mobile Erste-Hilfe-Endgerät sendet.

9. Verfahren zum intelligenten und rapiden Erwerb des AED, um an der Erste-Hilfe-Umgebung anzukommen, gemäß Anspruch 1, wobei

beim Durchführen der Drittbereichsberechnung der AED-Suche die Fußgängerverkehrsdaten durch Verwenden folgender Schritte erworben werden:

Sammeln eines Bildvideos nahe dem verfügbaren AED innerhalb eines vorherigen Zeitraums vor einem Moment des Empfangens der Erste-Hilfe-Informationen;

Durchführen von Bewegungsextraktion an räumlichen Objektinformationen, die in dem Bildvideo gesammelt werden, um den Fußgängerfluss nahe dem verfügbaren AED zu beurteilen; und

Kennzeichnen einer Dichte des Fußgängerflusses und Nehmen einer erworbenen Fußgängerflusswahrscheinlichkeitsdichteverteilungssituation als die Fußgängerverkehrsdaten.

10. Verfahren zum intelligenten und rapiden Erwerb des AED, um an der Erste-Hilfe-Umgebung anzukommen, gemäß Anspruch 9, wobei

beim Durchführen der Drittbereichsberechnung der AED-Suche ein Zeitraum des vorherigen Zeitraums festgelegt ist, um innerhalb von 10-20 Minuten vor dem Moment des Empfangens der Erste-Hilfe-Informationen nahe dem verfügbaren AED zu sein;

der Reaktionsfestlegungsindex auf 95 % festgelegt ist;

das Bildvideo durch eine Kamera an dem verfügbaren AED aufgenommen wird;

die finale Reaktionswahrscheinlichkeit=die Fußgängerverkehrsdaten Y*durchschnittliche Fußgängerreaktionswahrscheinlichkeit Z; und

wenn Y*Z>95 % und die AED-Lieferzeit C weniger als die AED-Lieferzeit A und die AED-Lieferzeit B ist, die Ankunftslösung der Drittbereichsberechnung der AED-Suche als die zügigste AED-Ankunftslösung ausgegeben wird.

11. Verfahren zum intelligenten und rapiden Erwerb des AED, um an der Erste-Hilfe-Umgebung anzukommen, gemäß Anspruch 1, wobei

beim Durchführen der Drittbereichsberechnung der AED-Suche ein Prozess des Berechnens der finalen Reaktionswahrscheinlichkeit des Fußgängers gemäß den Fußgängerverkehrsdaten und einer durchschnittlichen Fußgängerreaktionswahrscheinlichkeit nahe dem verfügbaren AED innerhalb des vorherigen Zeitraums wie folgt ist:

Sammeln eines Bildvideos nahe dem verfügbaren AED innerhalb eines vorherigen Zeitraums vor einem Moment des Empfangens der Erste-Hilfe-Informationen;

Einrichten eines gestaffelten lokalen Gitters nahe dem verfügbaren AED; und Vorkonstruieren einer Fußgängerflussdichteverteilungsmatrix, um eine Verteilungsinformation des Fußgängerflusses in einem einheitlichen lokalen Gitter darzustellen;

Durchführen von Bewegungsextraktion an räumlichen Objektinformationen, die in dem Bildvideo gesammelt werden, durch Verwenden eines Bildfilterungsverfahrens, um eine räumliche Fußgängerflussinformation zu extrahieren;

Projizieren der extrahierten räumlichen Fußgängerflussinformation auf das lokale Gitter in einer Ebenenkoordinate durch Verwenden eines Bildraumprojektionsverfahrens, um eine Wahrscheinlichkeitsdichteverteilungssituation des Fußgängerflusses an jedem Gitter des lokalen Gitters durch eine Ebenenprojektionskennzeichnung darzustellen, die als die Fußgängerverkehrsdaten dient; und

Berechnen der finalen Reaktionswahrscheinlichkeit von AED-Reaktion durch Poisson-Verteilung der Fußgängerflusswahrscheinlichkeit an dem lokalen Gitter in der Ebenenkoordinate.

12. Verfahren zum intelligenten und rapiden Erwerb des AED, um an der Erste-Hilfe-Umgebung anzukommen, gemäß Anspruch 11, wobei

der Schritt des Durchführens von Bewegungsextraktion an den räumlichen Objektinformationen, die in dem Bildvideo gesammelt werden, durch Verwenden des Bildfilterungsverfahrens folgende Schritte verwendet:

Rekonstruieren, für das erworbene Bildvideo nahe dem verfügbaren AED, eines Hintergrundbildes ohne den Fußgängerfluss gemäß Pixeln mit sich langsam ändernden Pixelwerten auf einer Zeitleiste des Bildvideos und Aktualisieren eines Hintergrundmodells in Echtzeit; für die Pixel, die als zu dem Fußgängerfluss in einer Detektion des Fußgängerflusses gehörend beurteilt werden, Beibehalten eines ursprünglichen Hintergrundpixelwertes und kein Aktualisieren; und für Pixel, die als der Hintergrund beurteilt werden, Aktualisieren des Hintergrundmodells gemäß den folgenden Regeln: $B_{n+1}(i, j) = \alpha B_n(i, j) + (1 - \alpha)G(i, j)$;

wobei $\alpha \varepsilon (0,1)$ eine Aktualisierungsrate ist, $B_n(i, j)$ einen Pixelwert des Pixels des Hintergrundbildes darstellt, und $G(i, j)$ einen Pixelwert des Pixels in einem aktuellen Rahmen darstellt;

Durchführen, für kontinuierliche Videobilder, von kontinuierlicher Gaußscher Verteilungsdifferenzextraktion an einem n-ten Bild, um Hintergrundrauschen zu filtern und ein Hintergrundbild B zu erwerben; und

Differenzieren des Bildvideos durch Verwenden eines HIS-Raums eines Farbbildes als einen Grundraum für Bilddifferenzierung und Binarisieren des Bildes, das nach Differenzierung erworben wird, um den Fußgängerfluss zu detektieren, um die räumliche Fußgängerflussinformation zu extrahieren.

13. Intelligente AED-Vorrichtung zur Verwendung in dem Verfahren gemäß einem der Ansprüche 1 bis 10, wobei

die intelligente AED-Vorrichtung ein Hauptsteuermodul, ein Videosammelmodul, ein Leistungsmodul, ein Kommunikationsmodul, ein Speichermodul, ein Videoanzeigemodul und ein Alarmmodul umfasst;
das Hauptsteuermodul dazu konfiguriert ist, Informationszentralisierung, Speicherung, Analyse und Entscheidungsfindung durchzuführen;
das Videosammelmodul dazu konfiguriert ist, ein Videobild nahe einem intelligenten AED zu sammeln;
das Leistungsmodul dazu konfiguriert ist, anderen Modulen Leistung zuzuführen;
das Speichermodul dazu konfiguriert ist, AED-Betriebsinformationen, Videobildinformationen und Detektionsinformationen zu speichern;
das Kommunikationsmodul für Kommunikation mit einer Erste-Hilfe-Cloud-Plattform verantwortlich ist;
das Videoanzeigemodul dazu konfiguriert ist, den optimalen Weg und die zu verbrauchenden Zeitinformationen entsprechend einer zügigsten AED-Ankunftslösung anzuzeigen; und
das Alarmmodul dazu konfiguriert ist, einen Alarm zu geben, wenn AED-Lieferzeit C als die zügigste AED-Ankunftslösung empfangen wird.

14. System zum intelligenten und rapiden Erwerb eines AED, um an der Erste-Hilfe-Umgebung anzukommen, zur Verwendung in dem Verfahren gemäß einem der Ansprüche 1 bis 10, wobei

das System ein mobiles Erste-Hilfe-Endgerät, die intelligente AED-Vorrichtung gemäß Anspruch 13 und eine Erste-Hilfe-Cloud-Plattform umfasst;
das mobile Erste-Hilfe-Endgerät eine Vorrichtung ist, die dazu konfiguriert ist, Notrufinformationen eines Erste-Hilfe-Ereignisses und einen geografischen Standort der Erste-Hilfe-Umgebung zu senden und den optimalen Weg und die zu verbrauchenden Zeitinformationen entsprechend einer zügigsten AED-Ankunftslösung zu empfangen und anzuzeigen; das mobile Erste-Hilfe-Endgerät mit der Erste-Hilfe-Cloud-Plattform auf eine entfernte drahtlose Weise kommuniziert;
die intelligente AED-Vorrichtung eine Vorrichtung ist, die zur externen Defibrillation verwendet wird; und
die Erste-Hilfe-Cloud-Plattform mit dem mobilen Erste-Hilfe-Endgerät und der intelligenten AED-Vorrichtung auf eine entfernte drahtlose Weise kommuniziert und die Erste-Hilfe-Cloud-Plattform dazu konfiguriert ist, Erstbereichsberechnung der AED-Suche, Zweitbereichsberechnung der AED-Suche und Drittbereichsberechnung der AED-Suche nach Empfangen der Notrufinformationen des Erste-Hilfe-Ereignisses, die durch das mobile Erste-Hilfe-Endgerät gesendet werden, durchzuführen, die zügigste AED-Ankunftslösung auszugeben und den optimalen Weg und die zu verbrauchenden Zeitinformationen entsprechend der zügigsten AED-Ankunftslösung einem AED-Lieferausführungspersonal über das mobile Erste-Hilfe-Endgerät oder die intelligente AED-Vorrichtung anzuzeigen.

15. Lesbares Speichermedium, wobei
das lesbare Speichermedium ein Steuerprogramm darauf speichert und das Steuerprogramm, wenn durch einen Prozessor ausgeführt, den Prozessor dazu veranlasst, das Verfahren gemäß einem der Ansprüche 1 bis 10 auszuführen.

**Revendications**

1. Procédé pour acquérir intelligemment et rapidement un DEA pour arriver sur une scène de premiers secours, comprenant :

l'acquisition d'informations d'appel de détresse d'un événement de premiers secours et d'un emplacement géographique de la scène de premiers secours, et la recherche d'un DEA disponible près de l'événement de premiers secours ;
la réalisation d'un calcul de première portée de recherche de DEA pour rechercher le DEA disponible le plus proche de l'événement de premiers secours et le verrou de ce DEA disponible, et le calcul d'un chemin optimal et d'un temps à écouler pour qu'un sauveteur aille de l'avant pour prendre le DEA et remettre le DEA en revenant, et l'émission en sortie d'un temps A de remise de DEA ;
la réalisation d'un calcul de deuxième portée de recherche de DEA pour rechercher le DEA disponible le plus proche de l'événement de premiers secours, et le calcul d'un chemin optimal et d'un temps à écouler pour qu'un bénévole pouvant être appelé ou un personnel de gestion de DEA près du DEA disponible acquière le DEA puis remette le DEA à la scène de premiers secours, et l'émission en sortie d'un temps B de remise de DEA ;

la réalisation d'un calcul de troisième portée de recherche de DEA pour rechercher le DEA disponible avec de la circulation piétonnière près de l'événement de premiers secours, et le calcul d'une probabilité de réponse finale de piétons selon des données de circulation piétonnière acquises et une probabilité de réponse piétonnière moyenne des piétons près du DEA disponible au cours d'une période précédente, et le calcul d'un chemin optimal et d'un temps à écouler pour que le piéton près du DEA disponible apporte une réponse finale pour acquérir le DEA puis remettre le DEA à la scène de premiers secours, et l'émission en sortie d'un temps C de remise de DEA ;

si la probabilité de réponse finale est supérieure à un indice de réglage de réponse, l'émission en sortie d'une solution d'arrivée avec une durée la plus courte parmi le temps A de remise de DEA, le temps B de remise de DEA et le temps C de remise de DEA en tant que solution d'arrivée de DEA la plus rapide ;

si la probabilité de réponse finale est inférieure à l'indice de réglage de réponse, l'émission en sortie d'une solution d'arrivée avec une durée la plus courte parmi le temps A de remise de DEA et le temps B de remise de DEA en tant que solution d'arrivée de DEA la plus rapide ; et

la définition d'un sauveteur, d'un bénévole pouvant être appelé, d'un personnel de gestion de DEA ou d'un piéton répondant correspondant à la solution d'arrivée de DEA la plus rapide en tant que personnel d'exécution de remise de DEA, et l'affichage du chemin optimal et des informations de temps à écouler correspondant à la solution d'arrivée de DEA la plus rapide au personnel d'exécution de remise de DEA, et l'acquisition, par le personnel d'exécution de remise de DEA, du DEA disponible à partir de l'emplacement du DEA disponible correspondant à la solution d'arrivée de DEA la plus rapide et la remise du DEA à la scène de premiers secours.

2. Procédé pour acquérir intelligemment et rapidement le DEA pour arriver sur la scène de premiers secours selon la revendication 1, dans lequel

le sauveteur initie un sauvetage en un clic sur un terminal mobile de premiers secours, après qu'une plateforme infonuagique de premiers secours a acquis les informations d'appel de détresse et l'emplacement géographique de l'événement, la plateforme infonuagique de premiers secours calcule le DEA disponible le plus proche de l'emplacement géographique de la scène de premiers secours et le DEA disponible avec la circulation piétonnière près de l'événement de premiers secours, dans lequel le DEA disponible est un équipement de DEA dans un état normal ; et

la plateforme infonuagique de premiers secours réalise le calcul de première portée de recherche de DEA, le calcul de deuxième portée de recherche de DEA et le calcul de troisième portée de recherche de DEA, et la plateforme infonuagique de premiers secours émet en sortie la solution d'arrivée de DEA la plus rapide au personnel d'exécution de remise de DEA, et transmet également le chemin optimal et les informations de temps à écouler correspondant à la solution d'arrivée de DEA la plus rapide, et des informations du personnel d'exécution de remise de DEA au terminal mobile de premiers secours.

3. Procédé pour acquérir intelligemment et rapidement le DEA pour arriver sur la scène de premiers secours selon la revendication 1, dans lequel

pour le calcul du chemin optimal et du temps à écouler du calcul de première portée de recherche de DEA, du calcul de deuxième portée de recherche de DEA et du calcul de troisième portée de recherche de DEA, le chemin optimal est acquis en utilisant un algorithme de chemin de recherche en graphique ;

lors de la réalisation du calcul de première portée de recherche de DEA, le temps A de remise de DEA = (chemin de départ optimal + chemin de retour optimal) / vitesse moyenne ;

lors de la réalisation du calcul de deuxième portée de recherche de DEA, le temps B de remise de DEA = (le chemin optimal du bénévole pouvant être appelé ou du personnel de gestion de DEA au DEA + le chemin optimal du DEA à la scène de premiers secours) / la vitesse moyenne ; et

lors de la réalisation du calcul de troisième portée de recherche de DEA, le temps C de remise de DEA = le chemin de optimal à partir du DEA à la scène de premiers secours / vitesse moyenne.

4. Procédé pour acquérir intelligemment et rapidement le DEA pour arriver sur la scène de premiers secours selon la revendication 3, dans lequel

le chemin optimal est acquis en utilisant les étapes suivantes :

la conversion d'un chemin de grille en un graphique pondéré ;

la création d'une matrice de distance entre un nœud et un point de départ selon le graphique pondéré, le réglage d'une valeur du point de départ sur 0, et le réglage d'éléments restants sur l'infini ;

la création d'une matrice de nœud parent du nœud, dans lequel l'initialisation est 0, indiquant l'absence de nœud parent ;

le fait de trouver un nœud avec un coût total d'évaluation le plus bas parmi les nœuds qui n'ont pas été complètement traversés dans la matrice de distance, et la définition du nœud comme un nœud actuel ; la définition du nœud actuel pour qu'il ait une distance infinie et le marquage du nœud actuel comme complètement traversé ; la détermination de nœuds non traversés parmi tous les nœuds adjacents du nœud actuel, le fait de juger si une distance pondérée d'un nœud adjacent est supérieure à un poids de bord du nœud actuel à un nœud suivant, et si c'est le cas, la mise à jour du nœud adjacent pour qu'il soit le nœud actuel ;

la répétition des étapes précédentes jusqu'à ce que le nœud actuel soit un point cible ; et

l'acquisition du chemin optimal en complétant un retour arrière du nœud parent selon la matrice de nœud parent.

5. Procédé pour acquérir intelligemment et rapidement le DEA pour arriver sur la scène de premiers secours selon la revendication 1, dans lequel

après l'émission en sortie de la solution d'arrivée dans une troisième portée de recherche de DEA en tant que solution d'arrivée de DEA la plus rapide, la solution d'arrivée de DEA la plus rapide est envoyée au DEA disponible et une alarme est donnée par le DEA disponible ; le piéton près du DEA disponible répond rapidement pour prendre le DEA et remet le DEA à la scène de premiers secours selon des informations de navigation du chemin optimal sur un écran de DEA ; et si le piéton près du DEA disponible donnant l'alarme ne répond pas pour acquérir le DEA dans le temps N, la solution d'arrivée dans la durée la plus courte entre le temps A de remise de DEA et le temps B de remise de DEA est émise en sortie en tant que solution d'arrivée de DEA la plus rapide, dans lequel une somme du temps N plus le temps C de remise de DEA est supérieure au temps A de remise de DEA et est supérieure au temps B de remise de DEA.

6. Procédé pour acquérir intelligemment et rapidement le DEA pour arriver sur la scène de premiers secours selon la revendication 1, dans lequel

lorsque l'événement de premiers secours se produit, le sauveteur initie un appel de détresse en un clic sur un terminal mobile de premiers secours, et une plateforme infonuagique de premiers secours acquiert un positionnement et une situation spécifique de la scène, calcule le DEA disponible le plus proche de l'emplacement géographique de l'événement de premiers secours et verrouille le DEA disponible, et calcule le chemin optimal et le temps à écouler pour que le sauveteur prenne et remette le DEA ; et lorsqu'il est calculé que le temps A de remise de DEA dans une première portée de recherche de DEA est le plus court, la plateforme infonuagique de premiers secours envoie un emplacement géographique du DEA, un état d'équipement, un mode de remise, une carte d'itinéraire, un emplacement en temps réel et des informations de compte à rebours de remise à un terminal mobile du sauveteur, et le sauveteur va prendre le DEA et arrive sur la scène de premiers secours.

7. Procédé pour acquérir intelligemment et rapidement le DEA pour arriver sur la scène de premiers secours selon la revendication 1, dans lequel

lorsque l'événement de premiers secours se produit, le sauveteur initie un appel de détresse en un clic sur un terminal mobile de premiers secours, et une plateforme infonuagique de premiers secours acquiert un positionnement et une situation spécifique de la scène, calcule le DEA disponible le plus proche de l'emplacement géographique de l'événement de premiers secours, et recherche le bénévole pouvant être appelé et le personnel de gestion de DEA près du DEA disponible ; lorsqu'il est calculé que le temps B de remise de DEA dans une deuxième portée de recherche de DEA est le plus court, une demande d'événement est envoyée à un terminal mobile du bénévole pouvant être appelé ou du personnel de gestion du DAE et le DAE disponible est verrouillé en même temps, et le bénévole pouvant être appelé ou le personnel de gestion du DAE reçoit la demande d'événement de premiers secours sur le terminal mobile et gère la demande d'événement de premiers secours ; et le chemin optimal et le temps à écouler pour que le personnel acquière le DEA et remette le DEA à la scène de premiers secours sont calculés, le bénévole pouvant être appelé ou le personnel de gestion de DEA va prendre le DEA et arrive sur la scène de premiers secours, et en même temps, la plateforme infonuagique de premiers secours envoie l'emplacement géographique du DEA, un état d'équipement, un mode de remise, une carte d'itinéraire, un emplacement en temps réel et des informations de compte à rebours de remise au terminal mobile de premiers secours.

8. Procédé pour acquérir intelligemment et rapidement le DEA pour arriver sur la scène de premiers secours selon la revendication 1, dans lequel

lorsque l'événement de premiers secours se produit, le sauveteur initie un appel de détresse en un clic sur un terminal mobile de premiers secours, et une plateforme infonuagique de premiers secours acquiert un positionnement et une situation spécifique de la scène, et recherche le DEA disponible avec le plus grand nombre de circulation piétonnière près de l'événement de premiers secours ; lorsqu'il est calculé que le temps C de remise de DEA dans une troisième portée de recherche de DEA est le plus court, le DEA disponible donne une alarme sonore ou lumineuse, une navigation de positionnement d'événement de premiers secours et des informations de description d'un événement spécifique sont envoyées au DEA disponible, et une demande d'événement est présentée sur un écran de DEA disponible ; le piéton près du DEA disponible, après avoir remarqué l'alarme du DEA disponible, ouvre une boîte extérieure de DEA pour acquérir le DEA et gère la demande d'événement de premiers secours ; le chemin optimal et le temps à écouler pour que le piéton acquière le DEA et remette le DEA à la scène après l'alarme automatique du DEA sont calculés, le piéton navigue vers la scène de premiers secours selon une invite d'informations d'équipement, et en même temps, la plateforme infonuagique de premiers secours envoie l'emplacement géographique du DEA, un état d'équipement, un mode de remise, une carte d'itinéraire, un emplacement en temps réel et des informations de compte à rebours de remise au terminal mobile de premiers secours.

9. Procédé pour acquérir intelligemment et rapidement le DEA pour arriver sur la scène de premiers secours selon la revendication 1, dans lequel
lors de la réalisation du calcul de troisième portée de recherche de DEA, les données de trafic piétonnier sont acquises en utilisant les étapes suivantes :

la collecte d'une image vidéo près du DEA disponible au cours d'une période précédente avant un moment de réception des informations de premiers secours ;
la réalisation d'une extraction de mouvement sur des informations d'objet spatiales collectées dans la vidéo d'image pour juger du flux de piétons près du DEA disponible ; et
la caractérisation d'une densité du flux de piétons, et la prise d'une situation de distribution de densité de probabilité d'écoulement piéton acquise en tant que données de circulation piétonnière.

10. Procédé pour acquérir intelligemment et rapidement le DEA pour arriver sur la scène de premiers secours selon la revendication 9, dans lequel

lors de la réalisation du calcul de troisième portée de recherche de DEA, une période temporelle de la période précédente est définie pour être comprise entre 10 et 20 minutes avant le moment de réception des informations de premiers secours près du DEA disponible ;
l'indice de réglage de réponse est réglé sur 95 % ;
la vidéo d'image est capturée par une caméra sur le DEA disponible ;
la probabilité de réponse finale = les données de trafic piétonnier Y * probabilité de réponse piétonnière moyenne Z ; et
lorsque Y * Z > 95 % et que le temps C de remise de DEA est inférieur au temps A de remise de DEA et au temps B de remise de DEA, la solution d'arrivée du calcul de troisième portée de recherche de DEA est émise en sortie en tant que solution d'arrivée de DEA la plus rapide.

11. Procédé pour acquérir intelligemment et rapidement le DEA pour arriver sur la scène de premiers secours selon la revendication 1, dans lequel
lors de la réalisation du calcul de troisième portée de recherche de DEA, un processus de calcul de la probabilité de réponse finale du piéton selon les données de trafic piétonnier et d'une probabilité de réponse piétonnière moyenne près du DEA disponible au cours de la période précédente est le suivant :

la collecte d'une image vidéo près du DEA disponible au cours d'une période précédente avant un moment de réception des informations de premiers secours ;
l'établissement d'une grille locale échelonnée près du DEA disponible ; et la préconstruction d'une matrice de distribution de densité de flux de piétons pour représenter des informations d'une distribution du flux de piétons dans une grille locale uniforme ;
la réalisation d'une extraction de mouvement sur des informations d'objet spatiales collectées dans la vidéo d'image en utilisant un procédé de filtrage d'image pour extraire des informations de flux de piétons spatiales ;
la projection des informations de flux de piétons spatiales extraites sur la grille locale dans une coordonnée de plan en utilisant un procédé de projection d'espace d'image, pour représenter une situation de distribution de densité de probabilité du flux de piétons sur chaque grille de la grille locale par une caractérisation de projection de plan, qui sert de données de circulation piétonnière ; et
le calcul de la probabilité de réponse finale de la réponse du DEA par distribution de Poisson de la probabilité de flux de piétons sur la grille locale dans la coordonnée du plan.

12. Procédé pour acquérir intelligemment et rapidement le DEA pour arriver sur la scène de premiers secours selon la revendication 11, dans lequel

l'étape de réalisation d'une extraction de mouvement sur les informations d'objet spatiales collectées dans la vidéo d'image en utilisant le procédé de filtrage d'image utilise les étapes suivantes :

la reconstruction, pour la vidéo d'image acquise près du DEA disponible, d'une image d'arrière-plan sans le flux de piétons selon des pixels avec des valeurs de pixels changeant lentement sur une chronologie de la vidéo d'image, et la mise à jour d'un modèle d'arrière-plan en temps réel ; pour les pixels jugés comme appartenant au flux de piétons dans une détection du flux de piétons, le maintien d'une valeur de pixel d'arrière-plan d'origine et l'absence de mise à jour ; et pour les pixels jugés comme l'arrière-plan, la mise à jour du modèle d'arrière-plan selon les règles suivantes : $B_{n+1}(i, j) = \alpha B_n(i, j) + (1 - \alpha)G(i, j)$ ;

dans lequel, $\alpha \in (0,1)$ est un taux de mise à jour, $B_n(i, j)$ représente une valeur de pixel du pixel de l'image d'arrière-plan, et $G(i, j)$ représente une valeur de pixel du pixel dans une trame courante ;

la réalisation, pour des images vidéo continues, d'une extraction différentielle de distribution gaussienne continue sur une $n^{ième}$ image pour filtrer le bruit d'arrière-plan et acquérir une image d'arrière-plan B ; et

la différenciation de la vidéo d'image en utilisant un espace HIS d'une image en couleur comme espace de base pour la différenciation d'image, et la binarisation de l'image acquise après la différenciation pour détecter le flux de piétons, pour extraire les informations de flux de piétons spatiales.

13. Dispositif DEA intelligent, destiné à être utilisé dans le procédé selon l'une quelconque des revendications 1 à 10, dans lequel

le dispositif DEA intelligent comprend un module de commande principal, un module de collecte vidéo, un module d'alimentation, un module de communication, un module de stockage, un module d'affichage vidéo et un module d'alarme ;

le module de commande principal est configuré pour réaliser une centralisation, le stockage, une analyse et une prise de décision des informations ;

le module de collecte vidéo est configuré pour collecter une image vidéo à proximité d'un DEA intelligent ;

le module d'alimentation est configuré pour fournir une alimentation à d'autres modules ;

le module de stockage est configuré pour stocker des informations de fonctionnement de DEA, des informations d'image vidéo et des informations de détection ;

le module de communication est chargé de communiquer avec une plateforme infonuagique de premiers secours ;

le module d'affichage vidéo est configuré pour afficher le chemin optimal et les informations de temps à écouler correspondant à une solution d'arrivée de DEA la plus rapide ; et

le module d'alarme est configuré pour donner une alarme lors de la réception du temps C de remise de DEA en tant que solution d'arrivée de DEA la plus rapide.

14. Système pour acquérir intelligemment et rapidement un DEA pour arriver sur une scène de premiers secours, destiné à être utilisé dans le procédé selon l'une quelconque des revendications 1 à 10, dans lequel

le système comprend un terminal mobile de premiers secours, le dispositif DEA intelligent selon la revendication 13, et une plateforme infonuagique de premiers secours ;

le terminal mobile de premiers secours est un dispositif configuré pour envoyer des informations d'appel de détresse d'un événement de premiers secours et d'un emplacement géographique de la scène de premiers secours, et pour recevoir et afficher le chemin optimal et les informations de temps à écouler correspondant à une solution d'arrivée de DEA la plus rapide ; le terminal mobile de premiers secours communique avec la plateforme infonuagique de premiers secours d'une manière sans fil à distance ;

le dispositif DEA intelligent est un dispositif utilisé pour la défibrillation externe ; et

la plateforme infonuagique de premiers secours communique avec le terminal mobile de premiers secours et le dispositif DEA intelligent d'une manière sans fil à distance, et la plateforme infonuagique de premiers secours est configurée pour réaliser un calcul de première portée de recherche de DEA, un calcul de deuxième portée de recherche de DEA et un calcul de troisième portée de recherche de DEA après avoir reçu les informations d'appel de détresse de l'événement de premiers secours envoyées par le terminal mobile de premiers secours, émettre en sortie la solution d'arrivée de DEA la plus rapide, et afficher le chemin optimal et les informations de temps à écouler correspondant à la solution d'arrivée de DEA la plus rapide à un personnel d'exécution de remise de DEA par l'intermédiaire du terminal mobile de premiers secours ou du dispositif DEA intelligent.

15. Support de stockage lisible, dans lequel

le support de stockage lisible stocke un programme de commande sur celui-ci, et le programme de commande, lorsqu'il est exécuté par un processeur, amène le processeur à exécuter le procédé selon l'une quelconque des revendications 1 à 10.

First-aid mobile terminal

Start

Initiating, by a rescuer, one-click distress call on the first-aid mobile terminal when a first-aid event occurs

First-aid cloud platform

Acquiring, by the first-aid cloud platform, a positioning and specific situation of a scene

Algorithm

Calculating, by an algorithm model, in real time to sort time of above three solutions for acquiring an AED and delivering the same to the scene, and output a fastest AED arrival solution

First-aid cloud platform, mobile terminal

Sending, by the first-aid cloud platform, information of the fastest AED arrival solution (such as a geographic location, an equipment state, a delivery manner, a route map, a real-time location, and delivery countdown information of the AED) to a mobile terminal of a rescuer

End

Fig. 1

Start

First-aid mobile terminal | Initiating, by a rescuer, one-click distress call on the first-aid mobile terminal when a first-aid event occurs

First-aid cloud platform | Acquiring, by the first-aid cloud platform, a positioning and specific situation of a scene

Algorithm | Measuring, by a system, an available AED closest to a geographical location of the first-aid event and lock the available AED (a definition of the available AED is that an AED equipment state is "normal")

Measuring, by the system, an optimal path and a to-be-consumed time for a rescuer to take and deliver the AED back and forth

First-aid cloud platform, mobile terminal | Sending, by the first-aid cloud platform, information such as a geographic location, an equipment state, a delivery manner, a route map, a real-time location, and delivery countdown information of the AED to the first-aid mobile terminal

Going, by the rescuer, to take the AED and arrive at the first-aid scene

End

Fig. 2

31

2. First-aid cloud platform

3. Computing

4. Feeding back to-be-consumed time for delivering the AED

Calculating time for taking and delivering an AED back and forth

AED Search for and lock the AED

AED

Local grid

Location where the first-aid event occurs

Return

Outbound

Rescuer

Global grid

1. Declaring a first-aid event by a first-aid mobile terminal

Sending a first-aid request

Fig. 3

Fig. 4

Fig. 5

Start

Initiating, by a rescuer, one-click distress call on a first-aid mobile terminal when a first-aid event occurs

Acquiring, by a first-aid cloud platform, a positioning and specific situation of a scene

Searching for a nearest available AED and locking the AED (searching for the AED within a third range needs to meet two conditions: 1. the AED with the highest number of pedestrian traffic near the equipment in the past 15 minutes near a first-aid scene may not necessarily be the closest AED; and 2. an equipment state is "available")

Giving a sound or light alarm by AED equipment

Sending, by a system, positioning navigation and specific event description information of the first-aid event to the AED, and display an event request on an AED screen

Opening, by a pedestrian near the available AED, after noticing the available AED alarm, an AED outer box to acquire the AED and handle the first-aid event request

Measuring, by the system, an optimal path and a to-be-consumed time for the pedestrian to acquire the AED and deliver the same to the scene after the AED gives an alarm by itself

Navigating, by the pedestrian, to the first-aid scene according to an equipment information prompt

End

Sending, by the first-aid cloud platform, information such as a geographic location, an equipment state, a delivery manner, a route map, a real-time location, and delivery countdown information of the AED to the first-aid mobile terminal

Fig. 6

Fig. 7

Fig. 8

Start

Declare a first-aid event by a mobile terminal

Handle the event on a backend

Measuring an AED search first-range through an algorithm

Measuring an AED search second-range through an algorithm

Measuring an AED search third-range through an algorithm

Outputting AED delivery time A

Outputting AED delivery time B

Outputting AED delivery time C and a pedestrian response probability

Sort the delivery time A, B and C

When the probability is greater than 95% and whether the time C is less than the time A and the time B

No → The solution is invalid → End

Yes

A>B>C

A>B

B>A

Whether the time A, B and C is less than 3 minutes

Yes

No

Outputting, by a system, a fastest AED arrival solution-third range

Outputting, by the system, the fastest AED arrival solution-second range

Outputting, by the system, the fastest AED arrival solution-first range

Outputting, by the system, a conclusion: the AED cannot arrive at a scene in a timely manner

Whether the pedestrian acquires the AED within N seconds

Yes → End

No

Fig. 9

Local grid

Fig. 10

Fig. 11

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202311389063
- WO 2020055676 A1
- WO 2018069383 A1
- CA 3154933 A1